# EUROPEAN PATENT SPECIFICATION

(11) **EP 2 612 869 B1**
(45) Date of publication and mention of the grant of the patent: **14.01.2015**
(21) Application number: 12195313.7
(22) Date of filing: 24.07.2008
(51) Int. Cl.: C07K 16/18, A61K 39/395, A61P 35/00, G01N 33/574

(54) **Ed-a antigen of fibrinogen that is associated with lymphomas**
Mit Lymphomen assoziiertes ED-A fibrinogen antigen
Antigène ED-A du fibrinogène associé aux lymphomes

(30) Priority: 25.07.2007 US 951765 P
(43) Date of publication of application: 10.07.2013
(62) Divisional of application: 08807180.8
(73) Proprietor: Philogen S.p.A., 53100 Siena (IT)
(72) Inventor: Neri, Dario, 8093 Zurich (CH); Villa, Alessandra, 20127 Milano (IT); Trachsel, Eveline, 8093 Zurich (CH); Rybak, Jascha-Nikolai, 79618 Rheinfelden (Baden) (DE)
(74) Representative: Keirstead, Tanis Evelyne

(56) References cited:
- EP-A- 0 603 735
- SCHLIEMANN C ET AL: "Three clinical-stage tumor targeting antibodies reveal differential expression of oncofetal fibronectin and tenascin-C isoforms in human lymphoma", LEUKEMIA RESEARCH, NEW YORK,NY, US, vol. 33, no. 12, 1 December 2009 (2009-12-01), pages 1718-1722, XP026611698, ISSN: 0145-2126, DOI: 10.1016/J.LEUKRES.2009.06.025 [retrieved on 2009-07-22]
- KATO RINA ET AL: "A new type of antimetastatic peptide derived from fibronectin.", CLINICAL CANCER RESEARCH : AN OFFICIAL JOURNAL OF THE AMERICAN ASSOCIATION FOR CANCER RESEARCH JUL 2002, vol. 8, no. 7, July 2002 (2002-07), pages 2455-2462, XP002713215, ISSN: 1078-0432

### SEQUENCE LISTING

<110> Philogen S.p.A.
<120> An Antigen Associated With Lung Cancers And Lymphomas
<130> TEK/FP6743421
<140> EP
   <141> 2008-07-24
<150> EP08807180.8
   <151> 2008-07-24
<150> PCT/IB2008/002536 <151> 2008-07-24
<150> US 60/951,765
   <151> 2007-07-25
<160> 193
<170> PatentIn version 3.3
<210> 1
   <211> 118
   <212> PRT
   <213> Artificial sequence
<220>
   <223> Synthetic sequence: Amino acid sequence of the anti-ED-A antibody H1 heavy chain (VH)
<400> 1
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Arg
               20                  25                  30
   Arg Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Thr His Leu Tyr Leu Phe Asp Tyr Trp Gly Gln Gly Thr
               100                 105                 110
   Leu Val Thr Val Ser Ser
           115
<210> 2
   <211> 125
   <212> PRT
   <213> Artificial sequence
<220>
   <223> Synthetic sequence: Amino acid sequence of the anti-ED-A antibody H1 light chain (VL)
<400> 2
   Glu Ile Val Leu Thr Gln Ser Pro Gly Thr Leu Ser Leu Ser Pro Gly
   1 5 10 15
   Glu Lys Ala Thr Leu Ser Cys Arg Ala Ser Gln Ser Val Ser Ser Ala
   20 25 30
   Trp Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Met Arg Gly Arg Pro
   85 90 95
   Pro Thr Phe Gly Gln Gly Thr Lys Val Glu Ile Lys Ala Ala Ala Glu
   100 105 110
   Gln Lys Leu Ile Ser Glu Glu Asp Leu Asn Gly Ala Ala
   115 120 125
<210> 3
   <211> 3
   <212> PRT
   <213> Artificial sequence
<220>
   <223> Synthetic sequence: Amino acid sequence of the heavy chain CDR1 of anti-ED-A antibody H1
<400> 3
   Pro Arg Arg
   1
<210> 4
   <211> 6
   <212> PRT
   <213> Artificial sequence
<220>
   <223> Synthetic sequence: Amino acid sequence of the heavy chain CDR2 of anti-ED-A antibody H1
<400> 4
   Ser Gly Ser Gly Gly Ser
   1 5
<210> 5
   <211> 6
   <212> PRT
   <213> Artificial sequence
<220>
   <223> Synthetic sequence: Amino acid sequence of the heavy chain CDR3 of anti-ED-A antibody H1
<400> 5
   Ser Thr His Leu Tyr Leu
   1               5
<210> 6
   <211> 3
   <212> PRT
   <213> Artificial sequence
<220>
   <223> Synthetic sequence: Amino acid sequence of the light chain CDR1 of anti-ED-A antibody H1
<400> 6
   Ser Ala Trp
   1
<210> 7
   <211> 7
   <212> PRT
   <213> Artificial sequence
<220>
   <223> Synthetic sequence: Amino acid sequence of the light chain CDR2 of anti-ED-A antibody H1
<400> 7
   Gly Ala Ser Ser Arg Ala Thr
   1               5
<210> 8
   <211> 6
   <212> PRT
   <213> Artificial sequence
<220>
   <223> Synthetic sequence: Amino acid sequence of the light chain CDR3 of anti-ED-A antibody H1
<400> 8
   Met Arg Gly Arg Pro Pro
   1               5
<210> 9
<400> 9
   000
<210> 10
<400> 10
   000
<210> 11
   <211> 20
   <212> PRT
   <213> Artificial sequence
<220>
   <223> Synthetic sequence: Amino acid sequence of the anti-ED-A antibody H1 linker sequence
<400> 11
   Gly Gly Gly Gly Ser Gly Gly Gly Gly Ser Gly Gly Gly Gly Ser Gly
   1               5                   10                  15
   Gly Gly Gly Ser
               20
<210> 12
   <211> 354
   <212> DNA
   <213> Artificial sequence
<220>
   <223> Synthetic sequence: Nucleotide sequence of the anti-ED-A antibody H1 heavy chain (VH)
<400> 12
   gaggtgcagc tggtggagtc tgggggaggc ttggtacagc ctggggggtc cctgagactc     60
   tcctgtgcag cctctggatt cacctttagc ccgcggagga tgagctgggt ccgccaggct    120
   ccagggaagg ggctggagtg ggtctcagct attagtggta gtggtggtag cacatactac    180
   gcagactccg tgaagggccg gttcaccatc tccagagaca attccaagaa cacgctgtat    240
   ctgcaaatga acagcctgag agccgaggac acggccgtat attactgtgc gaaaagtact    300
   catttgtatc tttttgacta ctggggccag ggaaccctgg tcaccgtctc gagt          354
<210> 13
   <211> 387
   <212> DNA
   <213> Artificial sequence
<220>
   <223> Synthetic sequence: Nucleotide sequence of the anti-ED-A antibody H1 light chain (VL)
<400> 13
   gaaattgtgt tgacgcagtc tccaggcacc ctgtctttgt ctccagggga aaaagccacc      60
   ctctcctgca gggccagtca gagtgttagc tctgcgtggt tagcctggta ccagcagaaa     120
   cctggccagg ctcccaggct cctcatctat ggtgcatcca gcagggccac tggcatccca     180
   gacaggttca gtggcagtgg gtctgggaca gacttcactc tcaccatcag cagactggag     240
   cctgaagatt ttgcagtgta ttactgtcag cagatgcgtg gtcggccgcc gacgttcggc     300
   caagggacca aggtggaaat caaagcggcc gcagaacaaa aactcatctc agaagaggat     360
   ctgaatgggg ccgcatagac tgtgaaa                                         387
<210> 14
   <211> 60
   <212> DNA
   <213> Artificial sequence
<220>
   <223> Synthetic sequence: Nucleotide sequence of the anti-ED-A antibody H1 linker sequence
<400> 14
   ggcggtggag gttctggcgg cggtggcagt ggcggtggag gttccggggg tggaggatct 60
<210> 15
   <211> 17
   <212> PRT
   <213> Mus musculus
<400> 15
   Phe Leu Thr Thr Thr Pro Asn Ser Leu Leu Val Ser Trp Gln Ala Pro
   1               5                   10                  15
   Arg
<210> 16
   <211> 12
   <212> PRT
   <213> Mus musculus
<400> 16
   Ile Ala Trp Glu Ser Pro Gln Gly Gln Val Ser Arg
   1               5                   10
<210> 17
   <211> 52
   <212> DNA
   <213> Artificial sequence
<220>
   <223> Synthetic sequence: Partially degenerate primer
<220>
   <221> misc_feature
   <222> 27, 28, 30, 31, 33, 34
   <223> n is a or g or c or t
<400> 17
   ctggagcctg gcggacccag ctcatmnnmn nmnngctaaa ggtgaatcca ga 52
<210> 18
   <211> 58
   <212> DNA
   <213> Artificial sequence
<220>
   <223> Synthetic sequence: Partially degenerate primer
<220>
   <221> misc_feature
   <222> 28, 29, 31, 32, 34, 35
   <223> n is a or g or c or t
<400> 18
   ccaggtttct gctggtacca ggctaamnnm nnmnngctaa cactctgact ggccctgc 58
<210> 19
   <211> 26
   <212> DNA
   <213> Artificial sequence
<220>
   <223> Synthetic sequence: Primer LMB3long
<400> 19
   caggaaacag ctatgaccat gattac 26
<210> 20
   <211> 30
   <212> DNA
   <213> Artificial sequence
<220>
   <223> Synthetic sequence: Primer fdseqlong
<400> 20
   gacgttagta aatgaatttt ctgtatgagg 30
<210> 21
   <211> 118
   <212> PRT
   <213> Artificial sequence
<220>
   <223> Synthetic sequence: Amino acid sequence of the anti-ED-A antibody B2 VH domain
<400> 21
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ala Ala
               20                  25                  30
   Lys Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Thr His Leu Tyr Leu Phe Asp Tyr Trp Gly Gln Gly Thr
               100                 105                 110
   Leu Val Thr Val Ser Ser
           115
<210> 22
   <211> 125
   <212> PRT
   <213> Artificial sequence
<220>
   <223> Synthetic sequence: Amino acid sequence of the anti-ED-A antibody B2 VL domain
<400> 22
   Glu Ile Val Leu Thr Gln Ser Pro Gly Thr Leu Ser Leu Ser Pro Gly
   1 5 10 15
   Glu Lys Ala Thr Leu Ser Cys Arg Ala Ser Gln Ser Val Ser Val Ala
   20 25 30
   Phe Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Met Arg Gly Arg Pro
   85 90 95
   Pro Thr Phe Gly Gln Gly Thr Lys Val Glu Ile Lys Ala Ala Ala Glu
   100 105 110
   Gln Lys Leu Ile Ser Glu Glu Asp Leu Asn Gly Ala Ala
   115 120 125
<210> 23
   <211> 3
   <212> PRT
   <213> Artificial sequence
<220>
   <223> Synthetic sequence: Amino acid sequence of the heavy chain CDR1 of anti-ED-A antibody B2
<400> 23
   Ala Ala Lys
   1
<210> 24
<400> 24
   000
<210> 25
<400> 25
   000
<210> 26
   <211> 3
   <212> PRT
   <213> Artificial sequence
<220>
   <223> Synthetic sequence: Amino acid sequence of the light chain CDR1 of anti-ED-A antibody B2
<400> 26
   Val Ala Phe
   1
<210> 27
<400> 27
   000
<210> 28
   <211> 5
   <212> PRT
   <213> Artificial sequence
<220>
   <223> Synthetic sequence: Linker sequence of F8 diabody
<400> 28
   Gly Ser Ser Gly Gly
   1 5
<210> 29
   <211> 15
   <212> DNA
   <213> Artificial sequence
<220>
   <223> Synthetic sequence: Linker sequence of F8 diabody
<400> 29
   gggtccagtg gcggt 15
<210> 30
<400> 30
   000
<210> 31
   <211> 118
   <212> PRT
   <213> Artificial sequence
<220>
   <223> Synthetic sequence: Amino acid sequence of the anti-ED-A antibody G9 VH domain
<400> 31
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser His Met
               20                  25                  30
   Gln Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Thr His Leu Tyr Leu Phe Asp Tyr Trp Gly Gln Gly Thr
               100                 105                 110
   Leu Val Thr Val Ser Ser
           115
<210> 32
   <211> 125
   <212> PRT
   <213> Artificial sequence
<220>
   <223> Synthetic sequence: Amino acid sequence of the anti-ED-A antibody G9 VL domain
<400> 32
   Glu Ile Val Leu Thr Gln Ser Pro Gly Thr Leu Ser Leu Ser Pro Gly
   1               5                   10                  15
   Glu Lys Ala Thr Leu Ser Cys Arg Ala Ser Gln Ser Val Ser Thr Ala
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 33
   <211> 3
   <212> PRT
   <213> Artificial sequence
<220>
   <223> Synthetic sequence: Amino acid sequence of the heavy chain CDR1 of anti-ED-A antibody G9
<400> 33
   His Met Gln
   1
<210> 34
<400> 34
   000
<210> 35
<400> 35
   000
<210> 36
   <211> 3
   <212> PRT
   <213> Artificial sequence
<220>
   <223> Synthetic sequence: Amino acid sequence of the light chain CDR1 of anti-ED-A antibody G9
<400> 36
   Thr Ala Phe
   1
<210> 37
<400> 37
   000
<210> 38
<400> 38
   000
<210> 39
<400> 39
   000
<210> 40
<400> 40
   000
<210> 41
   <211> 118
   <212> PRT
   <213> Artificial sequence
<220>
   <223> Synthetic sequence: Amino acid sequence of the anti-ED-A antibody C5 VH domain
<400> 41
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Pro Ile
   20 25 30
   Thr Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 42
   <211> 125
   <212> PRT
   <213> Artificial sequence
<220>
   <223> Synthetic sequence: Amino acid sequence of the anti-ED-A antibody C5 VL domain
<400> 42
   Glu Ile Val Leu Thr Gln Ser Pro Gly Thr Leu Ser Leu Ser Pro Gly
   1               5                   10                  15
   Glu Lys Ala Thr Leu Ser Cys Arg Ala Ser Gln Ser Val Ser Leu His
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 43
   <211> 3
   <212> PRT
   <213> Artificial sequence
<220>
   <223> Synthetic sequence: Amino acid sequence of the heavy chain CDR1 of anti-ED-A antibody C5
<400> 43
   Pro Ile Thr
   1
<210> 44
<400> 44
   000
<210> 45
<400> 45
   000
<210> 46
   <211> 3
   <212> PRT
   <213> Artificial sequence
<220>
   <223> Synthetic sequence: Amino acid sequence of the light chain CDR1 of anti-ED-A antibody C5
<400> 46
   Leu His Phe
   1
<210> 47
<400> 47
   000
<210> 48
<400> 48
   000
<210> 49
<400> 49
   000
<210> 50
<400> 50
   000
<210> 51
   <211> 118
   <212> PRT
   <213> Artificial sequence
<220>
   <223> Synthetic sequence: Amino acid sequence of the anti-ED-A antibody D5 VH domain
<400> 51
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Val Met
               20                  25                  30
   Lys Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 52
   <211> 125
   <212> PRT
   <213> Artificial sequence
<220>
   <223> Synthetic sequence: Amino acid sequence of the anti-ED-A antibody D5 VL domain
<400> 52
   Glu Ile Val Leu Thr Gln Ser Pro Gly Thr Leu Ser Leu Ser Pro Gly
   1 5 10 15
   Glu Lys Ala Thr Leu Ser Cys Arg Ala Ser Gln Ser Val Ser Asn Ala
   20 25 30
   Phe Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Met Arg Gly Arg Pro
   85 90 95
   Pro Thr Phe Gly Gln Gly Thr Lys Val Glu Ile Lys Ala Ala Ala Glu
   100 105 110
   Gln Lys Leu Ile Ser Glu Glu Asp Leu Asn Gly Ala Ala
   115 120 125
<210> 53
   <211> 3
   <212> PRT
   <213> Artificial sequence
<220>
   <223> Synthetic sequence: Amino acid sequence of the heavy chain CDR1 of anti-ED-A antibody D5
<400> 53
   Val Met Lys
   1
<210> 54
<400> 54
   000
<210> 55
<400> 55
   000
<210> 56
   <211> 3
   <212> PRT
   <213> Artificial sequence
<220>
   <223> Synthetic sequence: Amino acid sequence of the light chain CDR1 of anti-ED-A antibody D5
<400> 56
   Asn Ala Phe
   1
<210> 57
<400> 57
   000
<210> 58
<400> 58
   000
<210> 59
<400> 59
   000
<210> 60
<400> 60
   000
<210> 61
   <211> 118
   <212> PRT
   <213> Artificial sequence
<220>
   <223> Synthetic sequence: Amino acid sequence of the anti-ED-A antibody E5 VH domain
<400> 61
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Gly
               20                  25                  30
   Ser Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Thr His Leu Tyr Leu Phe Asp Tyr Trp Gly Gln Gly Thr
               100                 105                 110
   Leu Val Thr Val Ser Ser
           115
<210> 62
   <211> 125
   <212> PRT
   <213> Artificial sequence
<220>
   <223> Synthetic sequence: Amino acid sequence of the anti-ED-A antibody E5 VL domain
<400> 62
   Glu Ile Val Leu Thr Gln Ser Pro Gly Thr Leu Ser Leu Ser Pro Gly
   1               5                   10                  15
   Glu Lys Ala Thr Leu Ser Cys Arg Ala Ser Gln Ser Val Ser Leu Ala
               20                  25                  30
   His Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 63
   <211> 3
   <212> PRT
   <213> Artificial sequence
<220>
   <223> Synthetic sequence: Amino acid sequence of the heavy chain CDR1 of anti-ED-A antibody E5
<400> 63
   Thr Gly Ser
   1
<210> 64
<400> 64
   000
<210> 65
<400> 65
   000
<210> 66
   <211> 3
   <212> PRT
   <213> Artificial sequence
<220>
   <223> Synthetic sequence: Amino acid sequence of the light chain CDR1 of anti-ED-A antibody E5
<400> 66
   Leu Ala His
   1
<210> 67
<400> 67
   000
<210> 68
<400> 68
   000
<210> 69
<400> 69
   000
<210> 70
<400> 70
   000
<210> 71
   <211> 118
   <212> PRT
   <213> Artificial sequence
<220>
   <223> Synthetic sequence: Amino acid sequence of the anti-ED-A antibody C8 VH domain
<400> 71
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Leu Gln
               20                  25                  30
   Thr Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Thr His Leu Tyr Leu Phe Asp Tyr Trp Gly Gln Gly Thr
               100                 105                 110
   Leu Val Thr Val Ser Ser
           115
<210> 72
   <211> 125
   <212> PRT
   <213> Artificial sequence
<220>
   <223> Synthetic sequence: Amino acid sequence of the anti-ED-A antibody C8 VL domain
<400> 72
   Glu Ile Val Leu Thr Gln Ser Pro Gly Thr Leu Ser Leu Ser Pro Gly
   1               5                   10                  15
   Glu Lys Ala Thr Leu Ser Cys Arg Ala Ser Gln Ser Val Ser Leu Pro
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 73
   <211> 3
   <212> PRT
   <213> Artificial sequence
<220>
   <223> Synthetic sequence: Amino acid sequence of the heavy chain CDR1 of anti-ED-A antibody C8
<400> 73
   Leu Gln Thr
   1
<210> 74
<400> 74
   000
<210> 75
<400> 75
   000
<210> 76
   <211> 3
   <212> PRT
   <213> Artificial sequence
<220>
   <223> Synthetic sequence: Amino acid sequence of the light chain CDR1 of anti-ED-A antibody C8
<400> 76
   Leu Pro Phe
   1
<210> 77
<400> 77
   000
<210> 78
<400> 78
   000
<210> 79
<400> 79
   000
<210> 80
<400> 80
   000
<210> 81
   <211> 118
   <212> PRT
   <213> Artificial sequence
<220>
   <223> Synthetic sequence: Amino acid sequence of the anti-ED-A antibody F8 VH domain
<400> 81
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Leu Phe
   20 25 30
   Thr Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 82
   <211> 125
   <212> PRT
   <213> Artificial sequence
<220>
   <223> Synthetic sequence: Amino acid sequence of the anti-ED-A antibody F8 VL domain
<400> 82
   Glu Ile Val Leu Thr Gln Ser Pro Gly Thr Leu Ser Leu Ser Pro Gly
   1 5 10 15
   Glu Lys Ala Thr Leu Ser Cys Arg Ala Ser Gln Ser Val Ser Met Pro
   20 25 30
   Phe Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Met Arg Gly Arg Pro
   85 90 95
   Pro Thr Phe Gly Gln Gly Thr Lys Val Glu Ile Lys Ala Ala Ala Glu
   100 105 110
   Gln Lys Leu Ile Ser Glu Glu Asp Leu Asn Gly Ala Ala
   115 120 125
<210> 83
   <211> 3
   <212> PRT
   <213> Artificial sequence
<220>
   <223> Synthetic sequence: Amino acid sequence of the heavy chain CDR1 of anti-ED-A antibody F8
<400> 83
   Leu Phe Thr
   1
<210> 84
<400> 84
   000
<210> 85
<400> 85
   000
<210> 86
   <211> 3
   <212> PRT
   <213> Artificial sequence
<220>
   <223> Synthetic sequence: Amino acid sequence of the light chain CDR1 of anti-ED-A antibody F8
<400> 86
   Met Pro Phe
   1
<210> 87
<400> 87
   000
<210> 88
<400> 88
   000
<210> 89
<400> 89
   000
<210> 90
<400> 90
   000
<210> 91
   <211> 118
   <212> PRT
   <213> Artificial sequence
<220>
   <223> Synthetic sequence: Amino acid sequence of the anti-ED-A antibody F1 VH domain
<400> 91
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Gln Ala
               20                  25                  30
   Arg Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 92
   <211> 125
   <212> PRT
   <213> Artificial sequence
<220>
   <223> Synthetic sequence: Amino acid sequence of the anti-ED-A antibody F1 VL domain
<400> 92
   Glu Ile Val Leu Thr Gln Ser Pro Gly Thr Leu Ser Leu Ser Pro Gly
   1               5                   10                  15
   Glu Lys Ala Thr Leu Ser Cys Arg Ala Ser Gln Ser Val Ser Ala Pro
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 93
   <211> 3
   <212> PRT
   <213> Artificial sequence
<220>
   <223> Synthetic sequence: Amino acid sequence of the heavy chain CDR1 of anti-ED-A antibody F1
<400> 93
   Gln Ala Arg
   1
<210> 94
<400> 94
   000
<210> 95
<400> 95
   000
<210> 96
   <211> 3
   <212> PRT
   <213> Artificial sequence
<220>
   <223> Synthetic sequence: Amino acid sequence of the light chain CDR1 of anti-ED-A antibody F1
<400> 96
   Ala Pro Phe
   1
<210> 97
<400> 97
   000
<210> 98
<400> 98
   000
<210> 99
<400> 99
   000
<210> 100
<400> 100
   000
<210> 101
   <211> 118
   <212> PRT
   <213> Artificial sequence
<220>
   <223> Synthetic sequence: Amino acid sequence of the anti-ED-A antibody B7 VH domain
<400> 101
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser His Phe
   20 25 30
   Asp Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 102
   <211> 125
   <212> PRT
   <213> Artificial sequence
<220>
   <223> Synthetic sequence: Amino acid sequence of the anti-ED-A antibody B7 VL domain
<400> 102
   Glu Ile Val Leu Thr Gln Ser Pro Gly Thr Leu Ser Leu Ser Pro Gly
   1 5 10 15
   Glu Lys Ala Thr Leu Ser Cys Arg Ala Ser Gln Ser Val Ser Leu Ala
   20 25 30
   Phe Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Met Arg Gly Arg Pro
   85 90 95
   Pro Thr Phe Gly Gln Gly Thr Lys Val Glu Ile Lys Ala Ala Ala Glu
   100 105 110
   Gln Lys Leu Ile Ser Glu Glu Asp Leu Asn Gly Ala Ala
   115 120 125

<210> 103
   <211> 3
   <212> PRT
   <213> Artificial sequence
<220>
   <223> Synthetic sequence: Amino acid sequence of the heavy chain CDR1 of anti-ED-A antibody B7
<400> 103
   His Phe Asp
   1
<210> 104
<400> 104
   000
<210> 105
<400> 105
   000
<210> 106
   <211> 3
   <212> PRT
   <213> Artificial sequence
<220>
   <223> Synthetic sequence: Amino acid sequence of the light chain CDR1 of anti-ED-A antibody B7
<400> 106
   Leu Ala Phe
   1
<210> 107
<400> 107
   000
<210> 108
<400> 108
   000
<210> 109
<400> 109
   000
<210> 110
<400> 110
   000
<210> 111
   <211> 118
   <212> PRT
   <213> Artificial sequence
<220>
   <223> Synthetic sequence: Amino acid sequence of the anti-ED-A antibody E8 VH domain
<400> 111
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Met
               20                  25                  30
   His Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Thr His Leu Tyr Leu Phe Asp Tyr Trp Gly Gln Gly Thr
               100                 105                 110
   Leu Val Thr Val Ser Ser
           115
<210> 112
   <211> 125
   <212> PRT
   <213> Artificial sequence
<220>
   <223> Synthetic sequence: Amino acid sequence of the anti-ED-A antibody E8 VL domain
<400> 112
   Glu Ile Val Leu Thr Gln Ser Pro Gly Thr Leu Ser Leu Ser Pro Gly
   1               5                   10                  15
   Glu Lys Ala Thr Leu Ser Cys Arg Ala Ser Gln Ser Val Ser Ser Ser
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 113
   <211> 3
   <212> PRT
   <213> Artificial sequence
<220>
   <223> Synthetic sequence: Amino acid sequence of the heavy chain CDR1 of anti-ED-A antibody E8
<400> 113
   Asp Met His
   1
<210> 114
<400> 114
   000
<210> 115
<400> 115
   000
<210> 116
   <211> 3
   <212> PRT
   <213> Artificial sequence
<220>
   <223> Synthetic sequence: Amino acid sequence of the light chain CDR1 of anti-ED-A antibody E8
<400> 116
   Ser Ser Phe
   1
<210> 117
   <211> 350
   <212> PRT
   <213> Mus musculus
<400> 117
   Asn Gly Leu Gly Pro Ser Lys Thr Lys Thr Ala Ser Pro Asp Gln Thr
   1               5                   10                  15
   Glu Met Thr Ile Glu Gly Leu Gln Pro Thr Val Glu Tyr Val Val Ser
               20                  25                  30
   Val Tyr Ala Gln Asn Arg Asn Gly Glu Ser Gln Pro Leu Val Gln Thr
           35                  40                  45
   Ala Val Thr Asn Ile Asp Arg Pro Lys Gly Leu Ala Phe Thr Asp Val
       50                  55                  60
   Asp Val Asp Ser Ile Lys Ile Ala Trp Glu Ser Pro Gln Gly Gln Val
   65                  70                  75                  80
   Ser Arg Tyr Arg Val Thr Tyr Ser Ser Pro Glu Asp Gly Ile Arg Glu
                   85                  90                  95
   Leu Phe Pro Ala Pro Asp Gly Glu Asp Asp Thr Ala Glu Leu Gln Gly
               100                 105                 110
   Leu Arg Pro Gly Ser Glu Tyr Thr Val Ser Val Val Ala Leu His Asp
           115                 120                 125
   Asp Met Glu Ser Gln Pro Leu Ile Gly Ile Gln Ser Thr Ala Ile Pro
       130                 135                 140
   Ala Pro Thr Asn Leu Lys Leu Ser Gln Val Thr Pro Thr Ser Phe Thr
   145                 150                 155                 160
   Ala Gln Trp Ile Ala Pro Ser Val Gln Leu Thr Gly Tyr Arg Val Arg
                   165                 170                 175
   Val Asn Pro Lys Glu Lys Thr Gly Pro Met Lys Glu Ile Asn Leu Ser
               180                 185                 190
   Pro Asp Ser Ser Ser Val Ile Val Ser Gly Leu Met Val Ala Thr Lys
   195 200 205
   Tyr Glu Val Ser Val Tyr Ala Leu Lys Asp Thr Leu Thr Ser Arg Pro
   210 215 220
   Ala Gln Gly Val Ile Thr Thr Leu Glu Asn Val Ser Pro Pro Arg Arg
   225 230 235 240
   Ala Arg Val Thr Asp Ala Thr Glu Thr Thr Ile Thr Ile Ser Trp Arg
   245 250 255
   Thr Lys Thr Glu Thr Ile Thr Gly Phe Gln Val Asp Ala Ile Pro Ala
   260 265 270
   Asn Gly Gln Thr Pro Val Gln Arg Ser Ile Ser Pro Asp Val Arg Ser
   275 280 285
   Tyr Thr Ile Thr Gly Leu Gln Pro Gly Thr Asp Tyr Lys Ile His Leu
   290 295 300
   Tyr Thr Leu Asn Asp Asn Ala Arg Ser Ser Pro Val Ile Ile Asp Ala
   305 310 315 320
   Ser Thr Ala Ile Asp Ala Pro Ser Asn Leu Arg Phe Leu Thr Thr Thr
   325 330 335
   Pro Asn Ser Leu Leu Val Ser Trp Gln Ala Pro Arg Ala Arg
   340 345 350
<210> 118
   <211> 90
   <212> PRT
   <213> Homo sapiens
<400> 118
   Asn Ile Asp Arg Pro Lys Gly Leu Ala Phe Thr Asp Val Asp Val Asp
   1               5                   10                  15
   Ser Ile Lys Ile Ala Trp Glu Ser Pro Gln Gly Gln Val Ser Arg Tyr
               20                  25                  30
   Arg Val Thr Tyr Ser Ser Pro Glu Asp Gly Ile His Glu Leu Phe Pro
           35                  40                  45
   Ala Pro Asp Gly Glu Glu Asp Thr Ala Glu Leu Gln Gly Leu Arg Pro
       50                  55                  60
   Gly Ser Glu Tyr Thr Val Ser Val Val Ala Leu His Asp Asp Met Glu
   65                  70                  75                  80
   Ser Gln Pro Leu Ile Gly Thr Gln Ser Thr
                   85                  90
<210> 119
   <211> 90
   <212> PRT
   <213> Mus musculus
<400> 119
   Asn Ile Asp Arg Pro Lys Gly Leu Ala Phe Thr Asp Val Asp Val Asp
   1               5                   10                  15
   Ser Ile Lys Ile Ala Trp Glu Ser Pro Gln Gly Gln Val Ser Arg Tyr
               20                  25                  30
   Arg Val Thr Tyr Ser Ser Pro Glu Asp Gly Ile Arg Glu Leu Phe Pro
           35                  40                  45
   Ala Pro Asp Gly Glu Asp Asp Thr Ala Glu Leu Gln Gly Leu Arg Pro
       50                  55                  60
   Gly Ser Glu Tyr Thr Val Ser Val Val Ala Leu His Asp Asp Met Glu
   65                  70                  75                  80
   Ser Gln Pro Leu Ile Gly Ile Gln Ser Thr
                   85                  90
<210> 120
   <211> 288
   <212> PRT
   <213> Artificial sequence
<220>
   <223> Synthetic sequence: Antigen (11A12) containing the ED-A domain of human fibronectin
<400> 120
   Met Arg Ser Tyr Arg Thr Glu Ile Asp Lys Pro Ser Gln Met Gln Val
   1               5                   10                  15
   Thr Asp Val Gln Asp Asn Ser Ile Ser Val Lys Trp Leu Pro Ser Ser
               20                  25                  30
   Ser Pro Val Thr Gly Tyr Arg Val Thr Thr Thr Pro Lys Asn Gly Pro
           35                  40                  45
   Gly Pro Thr Lys Thr Lys Thr Ala Gly Pro Asp Gln Thr Glu Met Thr
       50                  55                  60
   Ile Glu Gly Leu Gln Pro Thr Val Glu Tyr Val Val Ser Val Tyr Ala
   65                  70                  75                  80
   Gln Asn Pro Ser Gly Glu Ser Gln Pro Leu Val Gln Thr Ala Val Thr
                   85                  90                  95
   Asn Ile Asp Arg Pro Lys Gly Leu Ala Phe Thr Asp Val Asp Val Asp
               100                 105                 110
   Ser Ile Lys Ile Ala Trp Glu Ser Pro Gln Gly Gln Val Ser Arg Tyr
           115                 120                 125
   Arg Val Thr Tyr Ser Ser Pro Glu Asp Gly Ile His Glu Leu Phe Pro
       130                 135                 140
   Ala Pro Asp Gly Glu Glu Asp Thr Ala Glu Leu Gln Gly Leu Arg Pro
   145                 150                 155                 160
   Gly Ser Glu Tyr Thr Val Ser Val Val Ala Leu His Asp Asp Met Glu
                   165                 170                 175
   Ser Gln Pro Leu Ile Gly Thr Gln Ser Thr Ala Ile Pro Ala Pro Thr
               180                 185                 190
   Asp Leu Lys Phe Thr Gln Val Thr Pro Thr Ser Leu Ser Ala Gln Trp
           195                 200                 205
   Thr Pro Pro Asn Val Gln Leu Thr Gly Tyr Arg Val Arg Val Thr Pro
       210                 215                 220
   Lys Glu Lys Thr Gly Pro Met Lys Glu Ile Asn Leu Ala Pro Asp Ser
   225                 230                 235                 240
   Ser Ser Val Val Val Ser Gly Leu Met Val Ala Thr Lys Tyr Glu Val
                   245                 250                 255
   Ser Val Tyr Ala Leu Lys Asp Thr Leu Thr Ser Arg Pro Ala Gln Gly
               260                 265                 270
   Val Val Thr Thr Leu Glu Asn Val Arg Ser His His His His His His
           275                 280                 285
<210> 121
   <211> 867
   <212> DNA
   <213> Artificial sequence
<220>
   <223> Synthetic sequence: Nucleotide sequence of antigen (11A12)
<400> 121
   atgagatcct accgaacaga aattgacaaa ccatcccaga tgcaagtgac cgatgttcag 60
   gacaacagca ttagtgtcaa gtggctgcct tcaagttccc ctgttactgg ttacagagta 120
   accaccactc ccaaaaatgg accaggacca acaaaaacta aaactgcagg tccagatcaa 180
   acagaaatga ctattgaagg cttgcagccc acagtggagt atgtggttag tgtctatgct 240
   cagaatccaa gcggagagag tcagcctctg gttcagactg cagtaaccaa cattgatcgc 300
   cctaaaggac tggcattcac tgatgtggat gtcgattcca tcaaaattgc ttgggaaagc 360
   ccacaggggc aagtttccag gtacagggtg acctactcga gccctgagga tggaatccat 420
   gagctattcc ctgcacctga tggtgaagaa gacactgcag agctgcaagg cctcagaccg 480
   ggttctgagt acacagtcag tgtggttgcc ttgcacgatg atatggagag ccagcccctg 540
   attggaaccc agtccacagc tattcctgca ccaactgacc tgaagttcac tcaggtcaca 600
   cccacaagcc tgagcgccca gtggacacca cccaatgttc agctcactgg atatcgagtg 660
   cgggtgaccc ccaaggagaa gaccggacca atgaaagaaa tcaaccttgc tcctgacagc 720
   tcatccgtgg ttgtatcagg acttatggtg gccaccaaat atgaagtgag tgtctatgct 780
   cttaaggaca ctttgacaag cagaccagct cagggagttg tcaccactct ggagaatgtc 840
   agatctcatc accatcacca tcactaa 867
<210> 122
   <211> 10
   <212> PRT
   <213> Mus musculus
<400> 122
   His Tyr Gln Ile Asn Gln Gln Trp Glu Arg
   1 5 10
<210> 123
   <211> 9
   <212> PRT
   <213> Mus musculus
<400> 123
   Val Gly Asp Thr Tyr Glu Arg Pro Lys
   1 5
<210> 124
   <211> 17
   <212> PRT
   <213> Mus musculus
<400> 124
   His Ala Leu Gln Ser Ala Ser Ala Gly Ser Gly Ser Phe Thr Asp Val
   1 5 10 15
   Arg
<210> 125
   <211> 7
   <212> PRT
   <213> Mus musculus
<400> 125
   Ile Gly Asp Gln Trp Asp Lys
   1 5
<210> 126
   <211> 11
   <212> PRT
   <213> Mus musculus
<400> 126
   Thr Phe Tyr Gln Ile Gly Asp Ser Trp Glu Lys
   1 5 10
<210> 127
   <211> 16
   <212> PRT
   <213> Mus musculus
<400> 127
   Trp Lys Glu Ala Thr Ile Pro Gly His Leu Asn Ser Tyr Thr Ile Lys
   1 5 10 15
<210> 128
   <211> 14
   <212> PRT
   <213> Mus musculus
<400> 128
   Glu Ala Thr Ile Pro Gly His Leu Asn Ser Tyr Thr Ile Lys
   1 5 10
<210> 129
   <211> 21
   <212> PRT
   <213> Mus musculus
<400> 129
   Gly Leu Thr Pro Gly Val Ile Tyr Glu Gly Gln Leu Ile Ser Ile Gln
   1 5 10 15
   Gln Tyr Gly His Arg
   20
<210> 130
   <211> 12
   <212> PRT
   <213> Mus musculus
<400> 130
   Trp Ser Arg Pro Gln Ala Pro Ile Thr Gly Tyr Arg
   1 5 10
<210> 131
   <211> 19
   <212> PRT
   <213> Mus musculus
<400> 131
   Ser Asp Asn Val Pro Pro Pro Thr Asp Leu Gln Phe Val Glu Leu Thr
   1 5 10 15
   Asp Val Lys
<210> 132
   <211> 16
   <212> PRT
   <213> Mus musculus
<400> 132
   Val Thr Ile Met Trp Thr Pro Pro Asp Ser Val Val Ser Gly Tyr Arg
   1 5 10 15
<210> 133
   <211> 15
   <212> PRT
   <213> Mus musculus
<400> 133
   Val Glu Val Leu Pro Val Ser Leu Pro Gly Glu His Gly Gln Arg
   1 5 10 15
<210> 134
   <211> 18
   <212> PRT
   <213> Mus musculus
<400> 134
   Asn Thr Phe Ala Glu Ile Thr Gly Leu Ser Pro Gly Val Thr Tyr Leu
   1 5 10 15
   Phe Lys
<210> 135
   <211> 8
   <212> PRT
   <213> Mus musculus
<400> 135
   Val Phe Ala Val His Gln Gly Arg
   1 5
<210> 136
   <211> 10
   <212> PRT
   <213> Mus musculus
<400> 136
   Thr Val Leu Val Thr Trp Thr Pro Pro Arg
   1 5 10
<210> 137
   <211> 10
   <212> PRT
   <213> Mus musculus
<400> 137
   Gln Tyr Asn Val Gly Pro Leu Ala Ser Lys
   1 5 10
<210> 138
   <211> 16
   <212> PRT
   <213> Mus musculus
<400> 138
   Asn Leu Gln Pro Gly Ser Glu Tyr Thr Val Thr Leu Val Ala Val Lys
   1 5 10 15
<210> 139
   <211> 12
   <212> PRT
   <213> Mus musculus
<400> 139
   Ala Thr Gly Val Phe Thr Thr Leu Gln Pro Leu Arg
   1 5 10
<210> 140
   <211> 13
   <212> PRT
   <213> Mus musculus
<400> 140
   Leu Gly Val Arg Pro Ser Gln Gly Gly Glu Ala Pro Arg
   1 5 10
<210> 141
   <211> 28
   <212> PRT
   <213> Mus musculus
<400> 141
   Val Val Thr Pro Leu Ser Pro Pro Thr Asn Leu His Leu Glu Ala Asn
   1 5 10 15
   Pro Asp Thr Gly Val Leu Thr Val Ser Trp Glu Arg
   20 25
<210> 142
   <211> 10
   <212> PRT
   <213> Mus musculus
<400> 142
   Ser Thr Thr Pro Asp Ile Thr Gly Tyr Arg
   1 5 10
<210> 143
   <211> 17
   <212> PRT
   <213> Mus musculus
<400> 143
   Val Thr Trp Ala Pro Pro Pro Ser Ile Glu Leu Thr Asn Leu Leu Val
   1 5 10 15
   Arg
<210> 144
   <211> 27
   <212> PRT
   <213> Mus musculus
<400> 144
   Thr Gly Leu Asp Ser Pro Thr Gly Phe Asp Ser Ser Asp Ile Thr Ala
   1 5 10 15
   Asn Ser Phe Thr Val His Trp Val Ala Pro Arg
   20 25
<210> 145
   <211> 9
   <212> PRT
   <213> Mus musculus
<400> 145
   Ala Pro Ile Thr Gly Tyr Ile Ile Arg
   1 5
<210> 146
   <211> 11
   <212> PRT
   <213> Mus musculus
<400> 146
   His His Ala Glu His Ser Val Gly Arg Pro Arg
   1 5 10
<210> 147
   <211> 18
   <212> PRT
   <213> Mus musculus
<400> 147
   Glu Glu Ser Pro Pro Leu Ile Gly Gln Gln Ala Thr Val Ser Asp Ile
   1 5 10 15
   Pro Arg
<210> 148
   <211> 21
   <212> PRT
   <213> Mus musculus
<400> 148
   Ile Thr Tyr Gly Glu Thr Gly Gly Asn Ser Pro Val Gln Glu Phe Thr
   1 5 10 15
   Val Pro Gly Ser Lys
   20
<210> 149
   <211> 12
   <212> PRT
   <213> Mus musculus
<400> 149
   Ser Pro Val Gln Glu Phe Thr Val Pro Gly Ser Lys
   1 5 10
<210> 150
   <211> 24
   <212> PRT
   <213> Mus musculus
<400> 150
   Ser Thr Ala Thr Ile Asn Asn Ile Lys Pro Gly Ala Asp Tyr Thr Ile
   1 5 10 15
   Thr Leu Tyr Ala Val Thr Gly Arg
   20
<210> 151
   <211> 15
   <212> PRT
   <213> Mus musculus
<400> 151
   Gly Asp Ser Pro Ala Ser Ser Lys Pro Val Ser Ile Asn Tyr Lys
   1 5 10 15
<210> 152
   <211> 22
   <212> PRT
   <213> Mus musculus
<400> 152
   Thr Glu Ile Asp Lys Pro Ser Gln Met Gln Val Thr Asp Val Gln Asp
   1 5 10 15
   Asn Ser Ile Ser Val Arg
   20
<210> 153
   <211> 12
   <212> PRT
   <213> Mus musculus
<900> 153
<210> 154
   <211> 29
   <212> PRT
   <213> Mus musculus
<400> 154
   Thr Ala Ser Pro Asp Gln Thr Glu Met Thr Ile Glu Gly Leu Gln Pro
   1               5                   10                  15
   Thr Val Glu Tyr Val Val Ser Val Tyr Ala Gln Asn Arg
               20                  25
<210> 155
   <211> 22
   <212> PRT
   <213> Mus musculus
<400> 155
   Asn Gly Glu Ser Gln Pro Leu Val Gln Thr Ala Val Thr Thr Ile Pro
   1               5                   10                  15
   Ala Pro Thr Asn Leu Lys
               20
<210> 156
   <211> 19
   <212> PRT
   <213> Mus musculus
<400> 156
   Asn Gly Glu Ser Gln Pro Leu Val Gln Thr Ala Val Thr Asn Ile Asp
   1               5                   10                  15
   Arg Pro Lys
<210> 157
   <211> 12
   <212> PRT
   <213> Mus musculus
<400> 157
   Ile Ala Trp Glu Ser Pro Gln Gly Gln Val Ser Arg
   1               5                   10
<210> 158
   <211> 11
   <212> PRT
   <213> Mus musculus
<400> 158
   Val Thr Tyr Ser Ser Pro Glu Asp Gly Ile Arg
   1 5 10
<210> 159
   <211> 24
   <212> PRT
   <213> Mus musculus
<400> 159
   Phe Ser Gln Val Thr Pro Thr Ser Phe Thr Ala Gln Trp Ile Ala Pro
   1 5 10 15
   Ser Val Gln Leu Thr Gly Tyr Arg
   20
<210> 160
   <211> 9
   <212> PRT
   <213> Mus musculus
<400> 160
   Tyr Glu Val Ser Val Tyr Ala Leu Lys
   1 5
<210> 161
   <211> 24
   <212> PRT
   <213> Mus musculus
<400> 161
   Thr Lys Thr Glu Thr Ile Thr Gly Phe Gln Val Asp Ala Ile Pro Ala
   1 5 10 15
   Asn Gly Gln Thr Pro Val Gln Arg
   20
<210> 162
   <211> 22
   <212> PRT
   <213> Mus musculus
<400> 162
   Thr Glu Thr Ile Thr Gly Phe Gln Val Asp Ala Ile Pro Ala Asn Gly
   1 5 10 15
   Gln Thr Pro Val Gln Arg
   20
<210> 163
   <211> 14
   <212> PRT
   <213> Mus musculus
<400> 163
   Ser Tyr Thr Ile Thr Gly Leu Gln Pro Gly Thr Asp Tyr Lys
   1 5 10
<210> 164
   <211> 11
   <212> PRT
   <213> Mus musculus
<400> 164
   Ile His Leu Tyr Thr Leu Asn Asp Asn Ala Arg
   1 5 10
<210> 165
   <211> 19
   <212> PRT
   <213> Mus musculus
<400> 165
   Ser Ser Pro Val Ile Ile Asp Ala Ser Thr Ala Ile Asp Ala Pro Ser
   1 5 10 15
   Asn Leu Arg
<210> 166
   <211> 17
   <212> PRT
   <213> Mus musculus
<400> 166
   Phe Leu Thr Thr Thr Pro Asn Ser Leu Leu Val Ser Trp Gln Ala Pro
   1 5 10 15
   Arg
<210> 167
   <211> 7
   <212> PRT
   <213> Mus musculus
<400> 167
   Ile Thr Gly Tyr Ile Ile Lys
   1 5
<210> 168
   <211> 9
   <212> PRT
   <213> Mus musculus
<400> 168
   Tyr Glu Lys Pro Gly Ser Pro Pro Arg
   1               5
<210> 169
   <211> 17
   <212> PRT
   <213> Mus musculus
<400> 169
   Pro Tyr Leu Pro Asn Val Asp Glu Glu Val Gln Ile Gly His Val Pro
   1               5                   10                  15
   Arg
<210> 170
   <211> 15
   <212> PRT
   <213> Mus musculus
<400> 170
   Gly Val Thr Tyr Asn Ile Ile Val Glu Ala Leu Gln Asn Gln Arg
   1               5                   10                  15
<210> 171
   <211> 23
   <212> PRT
   <213> Mus musculus
<400> 171
   Arg Pro Gly Ala Ala Glu Pro Ser Pro Asp Gly Thr Thr Gly His Thr
   1               5                   10                  15
   Tyr Asn Gln Tyr Thr Gln Arg
               20
<210> 172
   <211> 118
   <212> PRT
   <213> Artificial sequence
<220>
   <223> Synthetic sequence: VH domain of anti-ED-A antibody H1
<400> 172
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Pro Arg
   20 25 30
   Arg Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 173
   <211> 118
   <212> PRT
   <213> Artificial sequence
<220>
   <223> Synthetic sequence: VH domain of anti-ED-A antibody B2
<400> 173
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ala Ala
               20                  25                  30
   Lys Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Thr His Leu Tyr Leu Phe Asp Tyr Trp Gly Gln Gly Thr
               100                 105                 110
   Leu Val Thr Val Ser Ser
           115
<210> 174
   <211> 118
   <212> PRT
   <213> Artificial sequence
<220>
   <223> Synthetic sequence: VH domain of anti-ED-A antibody C5
<400> 174
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Pro Ile
   20 25 30
   Thr Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 175
   <211> 118
   <212> PRT
   <213> Artificial sequence
<220>
   <223> Synthetic sequence: VH domain of anti-ED-A antibody D5
<400> 175
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Val Met
   20 25 30
   Lys Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 176
   <211> 118
   <212> PRT
   <213> Artificial sequence
<220>
   <223> Synthetic sequence: VH domain of anti-ED-A antibody E5
<400> 176
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Thr Gly
               20                  25                  30
   Ser Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Thr His Leu Tyr Leu Phe Asp Tyr Trp Gly Gln Gly Thr
               100                 105                 110
   Leu Val Thr Val Ser Ser
           115
<210> 177
   <211> 118
   <212> PRT
   <213> Artificial sequence
<220>
   <223> Synthetic sequence: VH domain of anti-ED-A antibody C8
<400> 177
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Leu Gln
   20 25 30
   Thr Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 178
   <211> 118
   <212> PRT
   <213> Artificial sequence
<220>
   <223> Synthetic sequence: VH domain of anti-ED-A antibody F8
<400> 178
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Leu Phe
   20 25 30
   Thr Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 179
   <211> 118
   <212> PRT
   <213> Artificial sequence
<220>
   <223> Synthetic sequence: VH domain of anti-ED-A antibody F1
<400> 179
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Gln Ala
   20 25 30
   Arg Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 180
   <211> 118
   <212> PRT
   <213> Artificial sequence
<220>
   <223> Synthetic sequence: VH domain of anti-ED-A antibody B7
<400> 180
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser His Phe
               20                  25                  30
   Asp Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Thr His Leu Tyr Leu Phe Asp Tyr Trp Gly Gln Gly Thr
               100                 105                 110
   Leu Val Thr Val Ser Ser
           115
<210> 181
   <211> 118
   <212> PRT
   <213> Artificial sequence
<220>
   <223> Synthetic sequence: VH domain of anti-ED-A antibody E8
<400> 181
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Asp Met
   20 25 30
   His Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 182
   <211> 118
   <212> PRT
   <213> Artificial sequence
<220>
   <223> Synthetic sequence: VH domain of anti-ED-A antibody G9
<400> 182
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser His Met
   20 25 30
   Gln Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ser Thr His Leu Tyr Leu Phe Asp Tyr Trp Gly Gln Gly Thr
   100 105 110
   Leu Val Thr Val Ser Ser
   115
<210> 183
   <211> 125
   <212> PRT
   <213> Artificial sequence
<220>
   <223> Synthetic sequence: VL domain of anti-ED-A antibody H1
<400> 183
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ala
   20 25 30
   Trp Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Met Arg Gly Arg Pro
   85 90 95
   Pro Thr Phe Gly Gln Gly Thr Lys Val Glu Ile Lys Ala Ala Ala Glu
   100 105 110
   Gln Lys Leu Ile Ser Glu Glu Asp Leu Asn Gly Ala Ala
   115 120 125
<210> 184
   <211> 125
   <212> PRT
   <213> Artificial sequence
<220>
   <223> Synthetic sequence: VL domain of anti-ED-A antibody B2
<400> 184
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Val Ala
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 185
   <211> 125
   <212> PRT
   <213> Artificial sequence
<220>
   <223> Synthetic sequence: VL domain of anti-ED-A antibody C5
<400> 185
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Leu His
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 186
   <211> 125
   <212> PRT
   <213> Artificial sequence
<220>
   <223> Synthetic sequence: VL domain of anti-ED-A antibody D5
<400> 186
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Asn Ala
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 187
   <211> 125
   <212> PRT
   <213> Artificial sequence
<220>
   <223> Synthetic sequence: VL domain of anti-ED-A antibody E5
<400> 187
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Leu Ala
               20                  25                  30
   His Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 188
   <211> 125
   <212> PRT
   <213> Artificial sequence
<220>
   <223> Synthetic sequence: VL domain of anti-ED-A antibody C8
<400> 188
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Leu Pro
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
   85 90 95
   Pro Thr Phe Gly Gln Gly Thr Lys Val Glu Ile Lys Ala Ala Ala Glu
   100 105 110
   Gln Lys Leu Ile Ser Glu Glu Asp Leu Asn Gly Ala Ala
   115 120 125
<210> 189
   <211> 125
   <212> PRT
   <213> Artificial sequence
<220>
   <223> Synthetic sequence: VL domain of anti-ED-A antibody F8
<400> 189
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Met Pro
   20 25 30
   Phe Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Met Arg Gly Arg Pro
   85 90 95
   Pro Thr Phe Gly Gln Gly Thr Lys Val Glu Ile Lys Ala Ala Ala Glu
   100 105 110
   Gln Lys Leu Ile Ser Glu Glu Asp Leu Asn Gly Ala Ala
   115 120 125
<210> 190
   <211> 125
   <212> PRT
   <213> Artificial sequence
<220>
   <223> Synthetic sequence: VL domain of anti-ED-A antibody F1
<400> 190
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ala Pro
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 191
   <211> 125
   <212> PRT
   <213> Artificial sequence
<220>
   <223> Synthetic sequence: VL domain of anti-ED-A antibody B7
<400> 191
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Leu Ala
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 192
   <211> 125
   <212> PRT
   <213> Artificial sequence
<220>
   <223> Synthetic sequence: VL domain of anti-ED-A antibody E8
<400> 192
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Met Arg Gly Arg Pro
                   85                  90                  95
   Pro Thr Phe Gly Gln Gly Thr Lys Val Glu Ile Lys Ala Ala Ala Glu
               100                 105                 110
   Gln Lys Leu Ile Ser Glu Glu Asp Leu Asn Gly Ala Ala
           115                 120                 125
<210> 193
   <211> 125
   <212> PRT
   <213> Artificial sequence
<220>
   <223> Synthetic sequence: VL domain of anti-ED-A antibody G9
<400> 193
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Thr Ala
   20 25 30
   Phe Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Met Arg Gly Arg Pro
   85 90 95
   Pro Thr Phe Gly Gln Gly Thr Lys Val Glu Ile Lys Ala Ala Ala Glu
   100 105 110
   Gln Lys Leu Ile Ser Glu Glu Asp Leu Asn Gly Ala Ala
   115 120 125

## Description

The present invention relates to the detection and treatment of lymphomas. The invention involves use of an antibody that binds the ED-A isoform of fibronectin, especially an antibody that binds domain ED-A of fibronectin.

Angiogenesis describes the growth of new blood vessels from existing blood vessels and is a rare event in the adult but is a characteristic feature of many diseases, including the growth of solid tumors. Angiogenesis is required for tumours to grow beyond a few millimetres in diameter and tumours can induce angiogenesis through the secretion of various growth factors, e.g. Vascular Endothelial Growth Factor (VEGF). The new blood vessels formed as the result of angiogenesis are referred to as the neovasculature of the tumour, and a vigorous neovasculature is a characteristic feature of an aggressive tumour.

Fibronectin (FN) is a glycoprotein and is widely expressed in a variety of normal tissues and body fluids. It is a component of the extracellular matrix (ECM), and plays a role in many biological processes, including cellular adhesion, cellular migration, haemostasis, thrombosis, wound healing, tissue differentiation and oncogenic transformation.

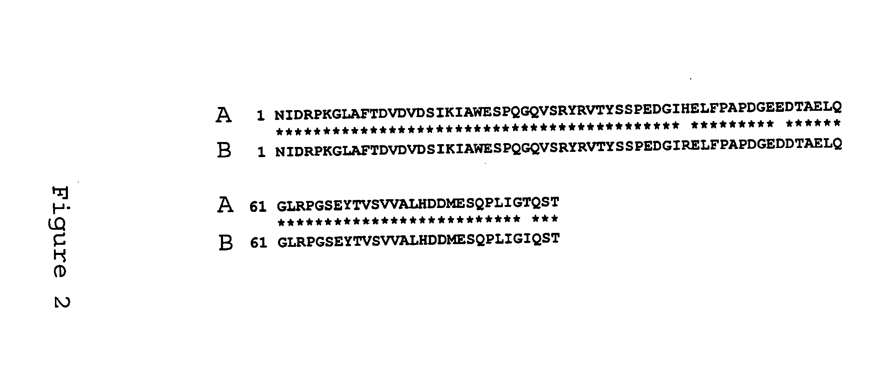

Different FN isoforms are generated by alternative splicing of three regions (ED-A, ED-B, IIICS) of the primary transcript FN pre-mRNA, a process that is modulated by cytokines and extracellular pH (Balza 1988; Carnemolla 1989; Borsi 1990; Borsi 1995). Fibronectin contains two type-III globular extra-domains which may undergo alternative splicing: ED-A and ED-B (ffrench-Constant 1995, Hynes 1990, Kaspar et al. 2006). The ED-As of mouse fibronectin and human fibronectin are 96.7% identical (only 3 amino acids differ between the two 90 amino acid sequences, see Figure 2).

Expression of the ED-A of fibronectin has been reported in tumour cells and in solid tumours at the mRNA level in breast cancer (Jacobs et al. 2002, Matsumoto et al. 1999) and liver cancer (Oyama et al. 1989, Tavian et al. 1994) and at the level of isolated protein in fibrosarcoma, rhabdomyosarcoma and melanoma (Borsi et al. 1987).

At the immunohistochemical level, the presence of ED-A has been detected in the extracellular matrix (ECM) of odontogenic tumours (Heikinheimo et al. 1991) and hepatocellular carcinoma (Koukoulis et al. 1995). In contrast, ED-A has been detected in the stroma of malignant breast neoplasms (Koukoulis et al. 1993), and in the blood vessels and basement membranes of well-differentiated renal cell carcinoma (Lohi et al. 1995). However, in less-differentiated renal cell carcinoma (Lohi et al. 1995) and papillary carcinoma of the thyroid (Scarpino et al. 1999) ED-A has been detected in the blood vessels, basement membranes and tumour stroma. The presence of ED-A in the vasculature of gliomas has also been reported (Borsi et al. 1998). Thus, the pattern of ED-A expression reported for different types of tumours is highly variable.

We show herein that ED-A is selectively expressed in the neovasculature of lymphomas. As tumour blood vessels are readily accessible for intravenously-administered therapeutic agents (Neri and Bicknell 2005, Rybak et al. 2006, Thorpe 2004, Trachsel and Neri 2006), binding molecules such as antibody molecules that bind the A-FN and/or the ED-A of fibronectin represent novel agents which may be used for the preparation of a medicament for the treatment of lymphomas.

Antibody-based method for the diagnosis and treatment of lymphoma have been suggested previously (EP0603735).

The therapy of tumour neo-vasculature (tumour vascular targeting) is a promising approach for the treatment of tumours. Tumour vascular targeting aims at disrupting the vasculature within the tumour itself, reducing blood flow to deprive the tumour of oxygen and nutrients, causing tumour cell death.

Disclosed herein are anti-ED-A antibodies which selectively recognize the new forming blood vessels of lymphomas.

The present invention is as set out in the claims.

The present invention provides an antibody that binds the Extra Domain-A (ED-A) isoform of fibronectin for use in a method of treating lymphoma, wherein the antibody is conjugated to a molecule that has biocidal or cytotoxic activity, or to a radioisotope.

The present invention provides an antibody that binds the ED-A isoform of fibronectin for use in an *in vivo* method of detecting or diagnosing lymphoma in a human or animal, wherein the antibody is labelled with a detectable label.

The present invention also provides an antibody that binds the ED-A isoform of fibronectin for use in a method of delivering a molecule conjugated to the antibody to the neovasculature of a lymphoma in a human or animal, wherein molecule has biocidal or cytotoxic activity, is a detectable label, or is a radioisotope.

The present invention also provides the use of an antibody that binds the ED-A isoform of fibronectin for the preparation of a medicament for delivery to a lymphoma of a molecule conjugated to the antibody, wherein molecule has biocidal or cytotoxic activity, is a detectable label, or is a radioisotope.

The present invention further provides an *in vitro* method of diagnosing lymphoma, wherein an antibody that binds the ED-A of fibronectin is used.

The invention also provides an antibody for use as set out above, wherein the *in vivo* method of diagnosing
a lymphoma in a human or animal comprises the steps of:
(a) administering to the human or animal an antibody molecule, which binds the ED-A isoform of fibronectin, and (b) determining the presence or absence of the antibody in the lymphatic system of the human or animal body;
   wherein localisation of the antibody to the lymphatic system in the human or animal indicates the presence of a lymphoma.

Further disclosed is a method of treating a lymphoma in an individual comprising administering to the individual a therapeutically effective amount of a medicament comprising a an antibody molecule, which binds the ED-A isoform of fibronectin. The present application also discloses a method of treating a lymphoma in an individual comprising administering to the individual a therapeutically effective amount of a medicament comprising an antibody molecule, which binds the ED-A of fibronectin.

Further disclosed is a method of delivering a molecule to the neovasculature of a lymphoma in a human or animal comprising administering to the human or animal an antibody molecule, which binds the ED-A isoform of fibronectin, wherein the antibody is conjugated to the molecule. Also disclosed is a method of delivering a molecule to the neovasculature of a lymphoma in a human or animal comprising administering to the human or animal an antibody molecule which binds the ED-A of fibronectin, wherein the antibody is conjugated to the molecule.

An antibody for use in the invention may be an antibody which binds the ED-A isoform of fibronectin and/or the ED-A of fibronectin, comprising one or more complementarity determining regions (CDRs) of antibody H1, B2, C5, D5, E5, C8, F8, F1, B7, E8 or G9, or variants thereof. Preferably, an antibody for use in the invention is an antibody which binds the ED-A isoform of fibronectin and/or the ED-A of fibronectin, comprising one or more complementarity determining regions (CDRs) of antibody B2, C5, D5, C8, F8, B7 or G9, or variants thereof. Most preferably, an antibody for use in the invention is an antibody which binds the ED-A isoform of fibronectin and/or the ED-A of fibronectin, comprising one or more complementarity determining regions (CDRs) of antibody F8 or variants thereof.

An antibody for use in the invention may comprise a set of H and/or L CDRs of antibody H1, B2, C5, D5, E5, C8, F8, F1, B7, E8 or G9, or a set of H and/or L CDRs of antibody H1, B2, C5, D5, E5, C8, F8, F1, B7, E8 or G9 with ten or fewer, e.g. one, two, three, four, or five, amino acid substitutions within the disclosed set.of H and/or L CDRs. Preferably, an antibody for use in the invention comprises a set of H and/or L CDRs of antibody B2, C5, D5, C8, F8, B7 or G9 with ten or fewer, e.g. one, two, three, four, or five, amino acid substitutions within the disclosed set of H and/or L CDRs. Preferably, an antibody for use in the invention comprises a set of H and/or L CDRs of antibody F8 with ten or fewer, e.g. one, two, three, four, or five, amino acid substitutions within the disclosed set of H and/or L CDRs.

Substitutions may potentially be made at any residue within the set of CDRs, and may be within CDR1, CDR2 and/or CDR3.

For example, an antibody for use in the invention may comprise one or more CDRs as described herein, e.g. a CDR3, and optionally also a CDR1 and CDR2 to form a set of CDRs.

An antibody for use in the invention may comprise a human antibody molecule. The antibody normally comprises an antibody VH and/or VL domain. VH domains of antibodies are also provided for use in the invention. Within each of the VH and VL domains are complementarity determining regions, ("CDRs"), and framework regions, ("FRs"). A VH domain comprises a set of HCDRs, and a VL domain comprises a set of LCDRs. An antibody molecule may comprise an antibody VH domain comprising a VH CDR1, CDR2 and CDR3 and a framework. It may alternatively or also comprise an antibody VL domain comprising a VL CDR1, CDR2 and CDR3 and a framework. The VH and VL domains and CDRs of antibodies H1, B2, C5, D5, E5, C8, F8, F1, B7, E8 and G9 are described herein. All VH and VL sequences, CDR sequences, sets of CDRs and sets of HCDRs and sets of LCDRs disclosed herein represent embodiments of and antibody for use in the invention. As described herein, a "set of CDRs" comprises CDR1, CDR2 and CDR3. Thus, a set of HCDRs refers to HCDR1, HCDR2 and HCDR3, and a set of LCDRs refers to LCDR1, LCDR2 and LCDR3. Unless otherwise stated, a "set of CDRs" includes HCDRs and LCDRs.

An antibody for use in the invention may comprise an antibody VH domain comprising complementarity determining regions HCDR1, HCDR2 and HCDR3 and a framework, wherein HCDR1 is SEQ ID NO: 3, 23, 33, 43, 53, 63, 73, 83, 93, 103 or 113, and wherein optionally HCDR2 is SEQ ID NO: 4 and/or HCDR3 is SEQ ID NO: 5. Preferably, the HCDR1 is SEQ ID NO: 23, 33, 43, 53, 73, 83 or 103. Most preferably, the HCDR1 is SEQ ID NO: 83.

Typically, a VH domain is paired with a VL domain to provide an antibody antigen-binding site, although as discussed further below a VH or VL domain alone may be used to bind antigen. Thus, a an antibody for use in the invention may further comprise an antibody VL domain comprising complementarity determining regions LCDR1, LCDR2 and LCDR3 and a framework, wherein LCDR1 is SEQ ID NO: 6, 26, 36, 46, 56, 66, 76, 86, 96, 106 or 116 and wherein optionally LCDR2 is SEQ ID NO: 7 and/or LCDR3 is SEQ ID NO: 8. Preferably, the LCDR1 is SEQ ID NO: 26, 36, 46, 56, 76, 86 or 106. Most preferably, the LCDR1 is SEQ ID NO: 86.

An antibody for use in the invention may be an isolated antibody molecule for the ED-A of fibronectin, comprising a VH domain and a VL domain, wherein the VH domain comprises a framework and a set of complementarity determining regions HCDR1, HCDR2 and HCDR3 and wherein the VL domain comprises complementarity determining regions LCDR1, LCDR2 and LCDR3 and a framework, and wherein
HCDR1 has amino acid sequence SEQ ID NO: 3, 23, 33, 43, 53, 63, 73, 83, 93, 103 or 113,
HCDR2 has amino acid sequence SEQ ID NO: 4,
HCDR3 has amino acid sequence SEQ ID NO: 5,
LCDR1 has amino acid sequence SEQ ID NO: 6, 26, 36, 46, 56, 66, 76, 86, 96, 106 or 116;
LCDR2 has amino acid sequence SEQ ID NO: 7; and
LCDR3 has amino acid sequence SEQ ID NO: 8.

One or more CDRs or a set of CDRs of an antibody may be grafted into a framework (e.g. human framework) to provide an antibody molecule for use in the invention. Framework regions may comprise human germline gene segment sequences. Thus, the framework may be germlined, whereby one or more residues within the framework are changed to match the residues at the equivalent position in the most similar human germline framework. An antibody for use in the invention may be an isolated antibody molecule having a VH domain comprising a set of HCDRs in a human germline framework, e.g. DP47. Normally the antibody also has a VL domain comprising a set of LCDRs, e.g. in a human germline framework. The human germline framework of the VL domain may be DPK22.

A VH domain may have amino acid sequence SEQ ID NO: 1, 21, 31, 41, 51, 61, 71, 81, 91, 101 or 111. Preferably, a VH domain has amino acid sequence SEQ ID NO: 21, 31, 41, 51, 71, 81 or 101. Most preferably, a VH domain for use in the invention has amino acid sequence SEQ ID NO: 81. A VL domain may have the amino acid SEQ ID NO: 2, 22, 32, 42, 52, 62, 72, 82, 92, 102 or 112. Preferably, a VL domain has amino acid SEQ ID NO: 22, 32, 42, 52, 72, 82 or 102. Most preferably, a VL domain has amino acid SEQ ID NO: 82.

An antibody for use in the invention may be a single chain Fv (scFv), comprising a VH domain and a VL domain joined via a peptide linker. The skilled person may select an appropriate length and sequence of linker, e.g. at least 5 or 10 amino acids in length, up to about 15, 20 or 25 amino acids in length. The linker may have the amino acid sequence GSSGG (SEQ ID NO: 28). The scFv may consist of or comprise amino acid sequence SEQ ID NO: 9.

Antibody molecules, are described in more detail elsewhere herein.

An antibody for use in the invention may be conjugated to a molecule that has biocidal or cytotoxic activity. Alternatively, a an antibody for use in the invention may be conjugated to a radioisotope. As a further alterative, an antibody for use in the invention may be labelled with a detectable label.

These and other aspects of the invention are described in further detail below.

### BRIEF DESCRIPTION OF THE DRAWINGS

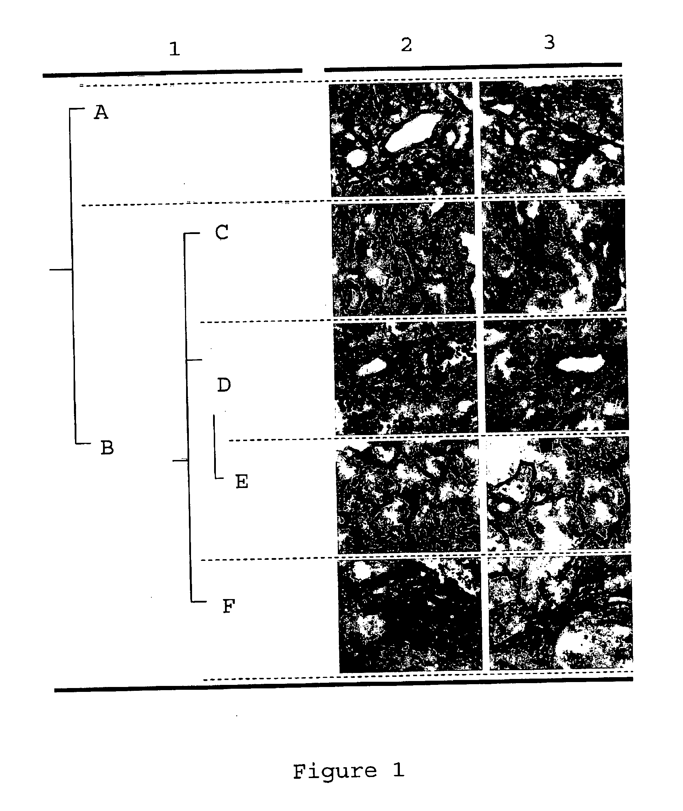

Figure 1: Shows the immunohistochemical staining of primary lung tumour sections with scFv anti-ED-A antibody D5. Column 1 indicates the classification of lung cancer: A: small cell lung cancer, B: non-small cell lung cancers, C: Squamous cell carcinoma, D: adenocarcinoma, E: bronchioalveolar carcinoma and F: large cell carcinoma. Columns 2 and 3: Show immunohistochemical detection of ED-A in tissue sections from lung tumours of the different subtypes. The tissue sections shown in columns 2 and 3 for each subtype were obtained from the same tumour specimen. Immunohistochemical staining (darker lines) revealed a strong vascular pattern of staining in primary tumour sections of both A: small cell lung cancer and B: non-small cell lung cancers (C: Squamous cell carcinoma, D: adenocarcinoma, E: bronchioalveolar carcinoma and F: large cell carcinoma).
Figure 2: Shows an alignment between A: the human ED-A (top sequence) and B: the mouse ED-A (bottom sequence). The asterisks indicate the amino acid positions where the amino acids of the human ED-A and the mouse ED-A are identical.

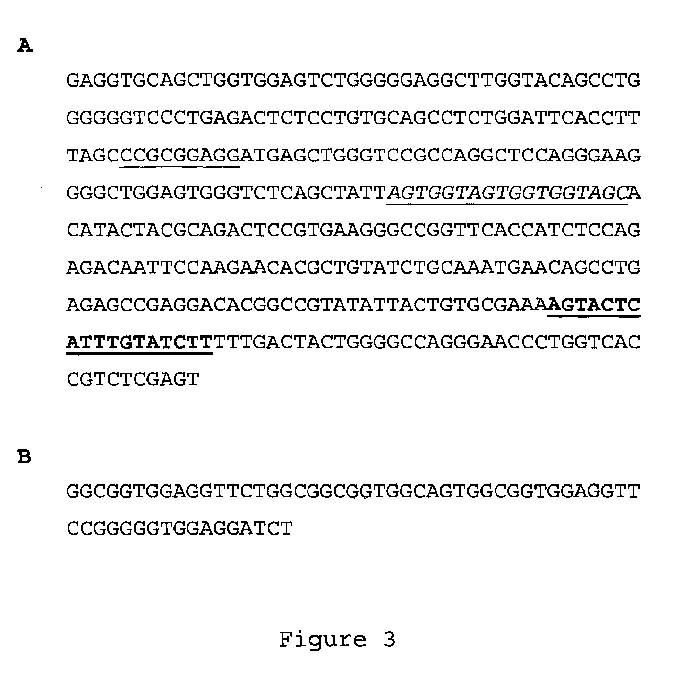

Figure 3 A: Shows the nucleotide sequence of the anti-ED-A antibody H1 heavy chain (VH) (SEQ ID NO: 12). The nucleotide sequence of the heavy chain CDR1 of anti-ED-A antibody H1 is underlined. The nucleotide sequence of the heavy chain CDR2 of the anti-ED-A antibody H1 is shown in *italics and underlined*. The nucleotide sequence of the heavy chain CDR3 of anti-ED-A antibody H1 is shown in **bold and underlined.** B: Shows the nucleotide sequence of the anti-ED-A antibody H1 linker sequence (SEQ ID NO: 14). C: Shows the nucleotide sequence of the anti-ED-A antibody H1 light chain (VL) (SEQ ID NO: 13). The nucleotide sequence of the light chain CDR1 of anti-ED-A antibody H1 is underlined. The nucleotide sequence of the light chain CDR2 of the anti-ED-A antibody H1 is shown in *italics and underlined*. The nucleotide sequence of the light chain CDR3 of anti-ED-A antibody H1 is shown in **bold and underlined.**

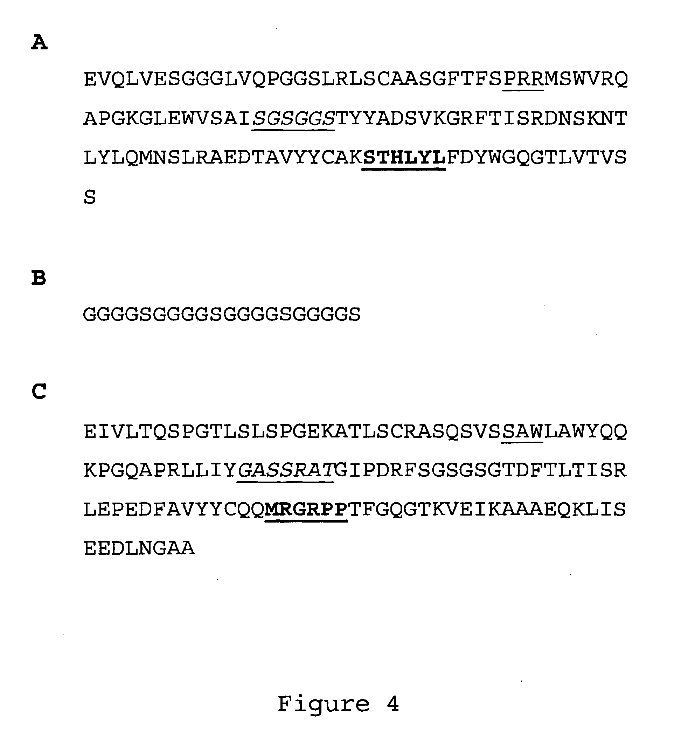

Figure 4 A: Shows the amino acid sequence of the anti-ED-A antibody H1 heavy chain (VH) (SEQ ID NO: 1). The amino acid sequence of the heavy chain CDR1 (SEQ ID NO: 3) of anti-ED-A antibody H1 is underlined. The amino acid sequence of the heavy chain CDR2 (SEQ ID NO: 4) of the anti-ED-A antibody H1 is shown in *italics and underlined.* The amino acid sequence of the heavy chain CDR3 (SEQ ID NO: 5) of anti-ED-A antibody H1 is shown in **bold and underlined.** B: Shows the amino acid sequence of the anti-ED-A antibody H1 linker sequence (SEQ ID NO: 11). C: Shows the amino acid sequence of the anti-ED-A antibody H1 light chain (VL) (SEQ ID NO: 2). The amino acid sequence of the light chain CDR1 (SEQ ID NO: 6) of anti-ED-A antibody H1 is underlined. The amino acid sequence of the light chain CDR2 (SEQ ID NO: 7) of the anti-ED-A antibody H1 is shown in *italics and underlined.* The amino acid sequence of the light chain CDR3 (SEQ ID NO: 8) of anti-ED-A antibody H1 is shown in **bold and underlined.** TERMINOLOGY

### Fibronectin

Fibronectin is an antigen subject to alternative splicing, and a number of alternative isoforms of fibronectin are known, as described elsewhere herein. Extra Domain-A (EDA or ED-A) is also known as ED, extra type III repeat A (EIIIA) or EDI. The sequence of human ED-A has been published by Kornblihtt et al. (1984), Nucleic Acids Res. 12, 5853-5868 and Paolella et al. (1988), Nucleic Acids Res. 16, 3545-3557. The sequence of human ED-A is also available on the SwissProt database as amino acids 1631-1720 (Fibronectin type-III 12; extra domain 2) of the amino acid sequence deposited under accession number P02751. The sequence of mouse ED-A is available on the SwissProt database as amino acids 1721-1810 (Fibronectin type-III 13; extra domain 2) of the amino acid sequence deposited under accession number P11276.

The ED-A isoform of fibronectin (A-FN) contains the Extra Domain-A (ED-A). The sequence of the human A-FN can be deduced from the corresponding human fibronectin precursor sequence which is available on the SwissProt database under accession number P02751. The sequence of the mouse A-FN can be deduced from the corresponding mouse fibronectin precursor sequence which is available on the SwissProt database under accession number P11276. The A-FN may be the human ED-A isoform of fibronectin. The ED-A may be the Extra Domain-A of human fibronectin.

ED-A is a 90 amino acid sequence which is inserted into fibronectin (FN) by alternative splicing and is located between domain 11 and 12 of FN (Borsi et al., 1987, J. Cell Biol., 104, 595-600). ED-A is mainly absent in the plasma form of FN but is abundant during embryogenesis, tissue remodelling, fibrosis, cardiac transplantation and solid tumour growth.

### Alternative splicing

Alternative splicing refers to the occurrence of different patterns of splicing of a primary RNA transcript of DNA to produce different mRNAs. After excision of introns, selection may determine which exons are spliced together to form the mRNA. Alternative splicing leads to production of different isoforms containing different exons and/or different numbers of exons. For example one isoform may comprise an additional amino acid sequence corresponding to one or more exons, which may comprise one or more domains.

### Lymphoma

This describes a type of cancer that involves cells of the immune system called lymophocytes and is characterised by an abnormal proliferation of these cells. There are many different subtypes of lymphomas and these can be grouped into two major categories: Hodgkin's lymphomas and non-Hodgkin's lymphomas. Hodgkin's lymphomas develop from a specific abnormal B lymphocyte lineage, while non-Hodgkin's lymphomas may derive from either abnormal B, T or NK cells and are distinguished by unique genetic markers. Burkitt's lymphoma is an example of a B-cell non-Hodgkin's lymphoma. A lymphoma as referred to herein may be a primary lymphoma. A lymphoma as referred to herein may be a Hodgkin's lymphoma or a non-Hodgkin's lymphoma. Preferably, a lymphoma as referred to herein is a primary Hodgkin's lymphoma or a primary non-Hodgkin's lymphoma.

### Primary tumour

This describes a tumour at the site where the tumour first arose (the primary site). Primary tumours sometimes spread from their original site (the primary site) to form secondary tumours (metastases) in other sites in the animal body and this spread is referred to as metastasis.

### Binding member

This describes one member of a pair of molecules that bind one another. The members of a binding pair may be naturally derived or wholly or partially synthetically produced. One member of the pair of molecules has an area on its surface, or a cavity, which binds to and is therefore complementary to a particular spatial and polar organization of the other member of the pair of molecules. Examples of types of binding pairs are antigen-antibody, biotin-avidin, hormone-hormone receptor, receptor-ligand, enzyme-substrate. The present invention is concerned with antigen-antibody type reactions.

A binding member normally comprises a molecule having an antigen-binding site. For example, a binding member may be an antibody molecule or a non-antibody protein that comprises an antigen-binding site.

An antigen binding site may be provided by means of arrangement of complementarity determining regions (CDRs) on non-antibody protein scaffolds such as fibronectin or cytochrome B etc. (Haan & Maggos, 2004; Koide 1998; Nygren 1997), or by randomising or mutating amino acid residues of a loop within a protein scaffold to confer binding specificity for a desired target. Scaffolds for engineering novel binding sites in proteins have been reviewed in detail by Nygren et al. (1997). Protein scaffolds for antibody mimics are disclosed in WO/0034784, in which the inventors describe proteins (antibody mimics) that include a fibronectin type III domain having at least one randomised loop. A suitable scaffold into which to graft one or more CDRs, e.g. a set of HCDRs, may be provided by any domain member of the immunoglobulin gene superfamily. The scaffold may be a human or non-human protein. An advantage of a non-antibody protein scaffold is that it may provide an antigen-binding site in a scaffold molecule that is smaller and/or easier to manufacture than at least some antibody molecules. Small size of a binding member may confer useful physiological properties such as an ability to enter cells, penetrate deep into tissues or reach targets within other structures, or to bind within protein cavities of the target antigen. Use of antigen binding sites in non-antibody protein scaffolds is reviewed in Wess, 2004. Typical are proteins having a stable backbone and one or more variable loops, in which the amino acid sequence of the loop or loops is specifically or randomly mutated to create an antigen-binding site that binds the target antigen. Such proteins include the IgG-binding domains of protein A from *S. aureus,* transferrin, tetranectin, fibronectin (e.g. 10th fibronectin type III domain) and lipocalins. Other approaches include synthetic "Microbodies" (Selecore GmbH), which are based on cyclotides - small proteins having intra-molecular disulphide bonds.

In addition to antibody sequences and/or an antigen-binding site, a binding member may comprise other amino acids, e.g. forming a peptide or polypeptide, such as a folded domain, or to impart to the molecule another functional characteristic in addition to ability to bind antigen. Binding members may carry a detectable label, or may be conjugated to a toxin or a targeting moiety or enzyme (e.g. via a peptidyl bond or linker). For example, a binding member may comprise a catalytic site (e.g. in an enzyme domain) as well as an antigen binding site, wherein the antigen binding site binds to the antigen and thus targets the catalytic site to the antigen. The catalytic site may inhibit biological function of the antigen, e.g. by cleavage.

Although, as noted, CDRs can be carried by non-antibody scaffolds, the structure for carrying a CDR or a set of CDRs will generally be an antibody heavy or light chain sequence or substantial portion thereof in which the CDR or set of CDRs is located at a location corresponding to the CDR or set of CDRs of naturally occurring VH and VL antibody variable domains encoded by rearranged immunoglobulin genes. The structures and locations of immunoglobulin variable domains may be determined by reference to Kabat 1987, and updates thereof, now available on the Internet (at immuno.bme.nwu.edu or find "Kabat" using any search engine).

By CDR region or CDR, it is intended to indicate the hypervariable regions of the heavy and light chains of the immunoglobulin as defined by Kabat et al. (1987), (Kabat 1991a, and later editions). An antibody typically contains 3 heavy chain CDRs and 3 light chain CDRs. The term CDR or CDRs is used here in order to indicate, according to the case, one of these regions or several, or even the whole, of these regions which contain the majority of the amino acid residues responsible for the binding by affinity of the antibody for the antigen or the epitope which it recognizes.

Among the six short CDR sequences, the third CDR of the heavy chain (HCDR3) has a greater size variability (greater diversity essentially due to the mechanisms of arrangement of the genes which give rise to it). It can be as short as 2 amino acids although the longest size known is 26. Functionally, HCDR3 plays a role in part in the determination of the specificity of the antibody (Segal 1974; Amit 1986; Chothia 1987; Chothia 1989; Caton 1990; Sharon 1990a; Sharon 1990b; Kabat et al., 1991b).

### Antibody Molecule

This describes an immunoglobulin whether natural or partly or wholly synthetically produced. The term also covers any polypeptide or protein comprising an antibody antigen-binding site. It must be understood here that the invention does not relate to the antibodies in natural form, that is to say they are not in their natural environment but that they have been able to be isolated or obtained by purification from natural sources, or else obtained by genetic recombination, or by chemical synthesis, and that they can then contain unnatural amino acids as will be described later. Antibody fragments that comprise an antibody antigen-binding site include, but are not limited to, antibody molecules such as Fab, Fab', Fab'-SH, scFv, Fv, dAb, Fd; and diabodies.

It is possible to take monoclonal and other antibodies and use techniques of recombinant DNA technology to produce other antibodies or chimeric molecules that bind the target antigen. Such techniques may involve introducing DNA encoding the immunoglobulin variable region, or the CDRs, of an antibody to the constant regions, or constant regions plus framework regions, of a different immunoglobulin. See, for instance, EP-A-184187, GB 2188638A or EP-A-239400, and a large body of subsequent literature. A hybridoma or other cell producing an antibody may be subject to genetic mutation or other changes, which may or may not alter the binding specificity of antibodies produced.

As antibodies can be modified in a number of ways, the term "antibody molecule" should be construed as covering any binding member or substance having an antibody antigen-binding site with the required specificity and/or binding to antigen. Thus, this term covers antibody fragments and derivatives, including any polypeptide comprising an antibody antigen-binding site, whether natural or wholly or partially synthetic. Chimeric molecules comprising an antibody antigen-binding site, or equivalent, fused to another polypeptide (e.g. derived from another species or belonging to another antibody class or subclass) are therefore included. Cloning and expression of chimeric antibodies are described in EP-A-0120694 and EP-A-0125023, and a large body of subsequent literature.

Further techniques available in the art of antibody engineering have made it possible to isolate human and humanised antibodies. For example, human hybridomas can be made as described by Kontermann & Dubel (2001). Phage display, another established technique for generating antibodies has been described in detail in many publications such as WO92/01047 (discussed further below) and US patents US5969108, US5565332, US5733743, US5858657, US5871907, US5872215, US5885793, US5962255, US6140471, US6172197, US6225447, US6291650, US6492160, US6521404 and Kontermann & Dubel (2001). Transgenic mice in which the mouse antibody genes are inactivated and functionally replaced with human antibody genes while leaving intact other components of the mouse immune system, can be used for isolating human antibodies (Mendez 1997).

Synthetic antibody molecules may be created by expression from genes generated by means of oligonucleotides synthesized and assembled within suitable expression vectors, for example as described by Knappik et al. (2000) or Krebs et al. (2001).

It has been shown that fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment (Ward 1989; McCafferty 1990; Holt 2003), which consists of a VH or a VL domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird 1988; Huston 1988); (viii) bispecific single chain Fv dimers (WO9311161) and (ix) "diabodies", multivalent or multispecific fragments constructed by gene fusion (WO94/13804; Holliger 1993a). Fv, scFv or diabody molecules may be stabilized by the incorporation of disulphide bridges linking the VH and VL domains (Reiter 1996). Minibodies comprising a scFv joined to a CH3 domain may also be made (Hu 1996). Other examples of binding fragments are Fab', which differs from Fab fragments by the addition of a few residues at the carboxyl terminus of the heavy chain CHl domain, including one or more cysteines from the antibody hinge region, and Fab'-SH, which is a Fab' fragment in which the cysteine residue(s) of the constant domains bear a free thiol group.

Antibody fragments for use in the invention can be obtained starting from any of the antibody molecules described herein, e.g. antibody molecules comprising VH and/or VL domains or CDRs of any of antibodies described herein, by methods such as digestion by enzymes, such as pepsin or papain and/or by cleavage of the disulfide bridges by chemical reduction. In another manner, antibody fragments of the present invention may be obtained by techniques of genetic recombination likewise well known to the person skilled in the art or else by peptide synthesis by means of, for example, automatic peptide synthesizers such as those supplied by the company Applied Biosystems, etc., or by nucleic acid synthesis and expression.

Functional antibody fragments include any functional fragment whose half-life is increased by a chemical modification, especially by PEGylation, or by incorporation in a liposome.

A dAb (domain antibody) is a small monomeric antigen-binding fragment of an antibody, namely the variable region of an antibody heavy or light chain (Holt 2003). VH dAbs occur naturally in camelids (e.g. camel, Ilama) and may be produced by immunizing a camelid with a target antigen, isolating antigen-specific B cells and directly cloning dAb genes from individual B cells. dAbs are also producible in cell culture. Their small size, good solubility and temperature stability makes them particularly physiologically useful and suitable for selection and affinity maturation. A antibody may be a dAb comprising a VH or VL domain substantially as set out herein, or a VH or VL domain comprising a set of CDRs substantially as set out herein.

As used herein, the phrase "substantially as set out" refers to the characteristic(s) of the relevant CDRs of the VH or VL domain of antibodies described herein will be either identical or highly similar to the specified regions of which the sequence is set out herein. As described herein, the phrase "highly similar" with respect to specified region(s) of one or more variable domains, it is contemplated that from 1 to about 5, e.g. from 1 to 4, including 1 to 3, or 1 or 2, or 3 or 4, amino acid substitutions may be made in the CDR and/or VH or VL domain.

Bispecific or bifunctional antibodies form a second generation of monoclonal antibodies in which two different variable regions are combined in the same molecule (Holliger 1999). Their use has been demonstrated both in the diagnostic field and in the therapy field from their capacity to recruit new effector functions or to target several molecules on the surface of tumor cells. Where bispecific antibodies are to be used, these may be conventional bispecific antibodies, which can be manufactured in a variety of ways (Holliger 1993b), e.g. prepared chemically or from hybrid hybridomas, or may be any of the bispecific antibody fragments mentioned above. These antibodies can be obtained by chemical methods (Glennie 1987; Repp 1995) or somatic methods (Staerz 1986; Suresh 1986) but likewise by genetic engineering techniques which allow the heterodimerization to be forced and thus facilitate the process of purification of the antibody sought (Merchand 1998). Examples of bispecific antibodies include those of the BiTE^{™} technology in which the binding domains of two antibodies with different specificity can be used and directly linked via short flexible peptides. This combines two antibodies on a short single polypeptide chain. Diabodies and scFv can be constructed without an Fc region, using only variable domains, potentially reducing the effects of anti-idiotypic reaction.

Bispecific antibodies can be constructed as entire IgG, as bispecific Fab'2, as Fab'PEG, as diabodies or else as bispecific scFv. Further, two bispecific antibodies can be linked using routine methods known in the art to form tetravalent antibodies.

Bispecific diabodies, as opposed to bispecific whole antibodies, may also be particularly useful because they can be readily constructed and expressed in E.coli. Diabodies (and many other polypeptides such as antibody fragments) of appropriate binding specificities can be readily selected using phage display (WO94/13804) from libraries. If one arm of the diabody is to be kept constant, for instance, with a specificity directed against a target antigen, then a library can be made where the other arm is varied and an antibody of appropriate specificity selected. Bispecific whole antibodies may be made by alternative engineering methods as described in Ridgeway 1996.

Various methods are available in the art for obtaining antibodies against a target antigen. The antibodies may be monoclonal antibodies, especially of human, murine, chimeric or humanized origin, which can be obtained according to the standard methods well known to the person skilled in the art.

In general, for the preparation of monoclonal antibodies or their functional fragments, especially of murine origin, it is possible to refer to techniques which are described in particular in the manual "Antibodies" (Harlow and Lane 1988) or to the technique of preparation from hybridomas described by Kohler and Milstein, 1975.

Monoclonal antibodies can be obtained, for example, from an animal cell immunized against A-FN, or one of its fragments containing the epitope recognized by said monoclonal antibodies, e.g. a fragment comprising or consisting of ED-A, or a peptide fragment of ED-A. The A-FN, or one of its fragments, can especially be produced according to the usual working methods, by genetic recombination starting with a nucleic acid sequence contained in the cDNA sequence coding for A-FN or fragment thereof, by peptide synthesis starting from a sequence of amino acids comprised in the peptide sequence of the A-FN and/or fragment thereof.

Monoclonal antibodies can, for example, be purified on an affinity column on which A-FN or one of its fragments containing the epitope recognized by said monoclonal antibodies, e.g. a fragment comprising or consisting of ED-A or a peptide fragment of ED-A, has previously been immobilized. Monoclonal antibodies can be purified by chromatography on protein A and/or G, followed or not followed by ion-exchange chromatography aimed at eliminating the residual protein contaminants as well as the DNA and the LPS, in itself, followed or not followed by exclusion chromatography on Sepharose gel in order to eliminate the potential aggregates due to the presence of dimers or of other multimers. The whole of these techniques may be used simultaneously or successively.

### Antigen-binding site

This describes the part of a molecule that binds to and is complementary to all or part of the target antigen. In an antibody molecule it is referred to as the antibody antigen-binding site, and comprises the part of the antibody that binds to and is complementary to all or part of the target antigen. Where an antigen is large, an antibody may only bind to a particular part of the antigen, which part is termed an epitope. An antibody antigen-binding site may be provided by one or more antibody variable domains. An antibody antigen-binding site may comprise an antibody light chain variable region (VL) and an antibody heavy chain variable region (VH).

### Isolated

This refers to the state in which antibodies and binding members described herein, and nucleic acid encoding such antibodies and binding members, will generally be. Thus, binding members, VH and/or VL domains may be provided isolated and/or purified, e.g. from their natural environment, in substantially pure or homogeneous form, or, in the case of nucleic acid, free or substantially free of nucleic acid or genes of origin other than the sequence encoding a polypeptide with the required function. Isolated members and isolated nucleic acid will be free or substantially free of material with which they are naturally associated such as other polypeptides or nucleic acids with which they are found in their natural environment, or the environment in which they are prepared (e.g. cell culture) when such preparation is by recombinant DNA technology practised i*n vitro* or *in vivo*. Members and nucleic acid may be formulated with diluents or adjuvants and still for practical purposes be isolated - for example the members will normally be mixed with gelatin or other carriers if used to coat microtitre plates for use in immunoassays, or will be mixed with pharmaceutically acceptable carriers or diluents when used in diagnosis or therapy. Antibodies may be glycosylated, either naturally or by systems of heterologous eukaryotic cells (e.g. CHO or NS0 (ECACC 85110503) cells, or they may be (for example if produced by expression in a prokaryotic cell) unglycosylated.

Heterogeneous preparations comprising antibody molecules may also be used in the invention. For example, such preparations may be mixtures of antibodies with full-length heavy chains and heavy chains lacking the C-terminal lysine, with various degrees of glycosylation and/or with derivatized amino acids, such as cyclization of an N-terminal glutamic acid to form a pyroglutamic acid residue.

One or more antibodies for an antigen, e.g. the A-FN or the ED-A of fibronectin, may be obtained by bringing into contact a library of antibodies and the antigen or a fragment thereof, e.g. a fragment comprising or consisting of ED-A or a peptide fragment of ED-A and selecting one or more antibodies of the library able to bind the antigen.

An antibody library may be screened using Iterative Colony Filter Screening (ICFS). In ICFS, bacteria containing the DNA encoding several binding specificities are grown in a liquid medium and, once the stage of exponential growth has been reached, some billions of them are distributed onto a growth support consisting of a suitably pre-treated membrane filter which is incubated until completely confluent bacteriae colonies appear. A second trap substrate consists of another membrane filter, pre-humidified and covered with the desired antigen.

The trap membrane filter is then placed onto a plate containing a suitable culture medium and covered with the growth filter with the surface covered with bacterial colonies pointing upwards. The sandwich thus obtained is incubated at room temperature for about 16 h. It is thus possible to obtain the expression of the genes encoding antibody fragments scFv having a spreading action, so that those fragments binding specifically with the antigen which is present on the trap membrane are trapped. The trap membrane is then treated to point out bound antibody fragments scFv with colorimetric techniques commonly used to this purpose.

The position of the coloured spots on the trap filter allows to go back to the corresponding bacterial colonies which are present on the growth membrane and produced the antibody fragments trapped. Such colonies are gathered and grown and the bacteria-a few millions of them are distributed onto a new culture membrane repeating the procedures described above. Analogous cycles are then carried out until the positive signals on the trap membrane correspond to single positive colonies, each of which represents a potential source of monoclonal antibody fragments directed against the antigen used in the selection. ICFS is described in e.g. WO0246455. A library may also be displayed on particles or molecular complexes, e.g. replicable genetic packages such bacteriophage (e.g. T7) particles, or other *in vitro* display systems, each particle or molecular complex containing nucleic acid encoding the antibody VH variable domain displayed on it, and optionally also a displayed VL domain if present. Phage display is described in WO92/01047 and e.g. US patents US5969108, US5565332, US5733743, US5858657, US5871907, US5872215, US5885793, US5962255, US6140471, US6172197, US6225447, US6291650, US6492160 and US6521404.

Following selection of antibodies able to bind the antigen and displayed on bacteriophage or other library particles or molecular complexes, nucleic acid may be taken from a bacteriophage or other particle or molecular complex displaying a said selected antibody. Such nucleic acid may be used in subsequent production of an antibody or an antibody VH or VL variable domain by expression from nucleic acid with the sequence of nucleic acid taken from a bacteriophage or other particle or molecular complex displaying a said selected antibody.

An antibody VH variable domain with the amino acid sequence of an antibody VH variable domain of a said selected antibody may be provided in isolated form, as may an antibody comprising such a VH domain.

Ability to bind the A-FN or the ED-A of fibronectin or other target antigen or isoform may be further tested, e.g. ability to compete with e.g. any one of anti-ED-A antibodies H1, B2, C5, D5, E5, C8, F8, F1, B7, E8 or G9 for binding to the A-FN or a fragment of the A-FN, e.g the ED-A of fibronectin.

An antibody for use in the invention may bind the A-FN and/or the ED-A of fibronectin specifically. An antibody for use in the invention may bind the A-FN and/or the ED-A of fibronectin with the same affinity as anti-ED-A antibody H1, B2, C5, D5, E5, C8, F8, F1, B7, E8 or G9, e.g. in scFv format, or with an affinity that is better. An antibody for use in the invention may bind the A-FN and/or the ED-A of fibronectin with a K_{D} of 3 x 10⁻⁸ M or an affinity that is better. Preferably, an antibody for use in the invention binds the A-FN and/or the ED-A of fibronectin with a K_{D} of 2 x 10⁻⁸ M or an affinity that is better. More preferably, anantibody for use in the invention binds the A-FN and/or the ED-A of fibronectin with a K_{D} of 1.7 x 10⁻⁸ M or an affinity that is better. Yet more preferably, an antibody for use in the invention binds the A-FN and/or the ED-A of fibronectin with a K_{D} of 1.4 x 10⁻⁸ M or an affinity that is better. Most preferably, a an antibody for use in the invention binds the A-FN and/or the ED-A of fibronectin with a K_{D} of 3 x 10⁻⁹ M or an affinity that is better.

An antibody for use in the present invention may bind to the same epitope on A-FN and/or the ED-A of fibronectin as anti-ED-A antibody H1, B2, C5, D5, E5, C8, F8, F1, B7, E8 or G9.

An antibody for use in the invention may not show any significant binding to molecules other than the A-FN and/or the ED-A of fibronectin. In particular the antibody may not bind other isoforms of fibronectin, for example the ED-B isoform and/or the IIICS isoform of fibronectin.

Variants of antibody molecules disclosed herein may be produced and used in the present invention. The techniques required to make substitutions within amino acid sequences of CDRs, antibody VH or VL domains and antibodies generally are available in the art. Variant sequences may be made, with substitutions that may or may not be predicted to have a minimal or beneficial effect on activity, and tested for ability to bind A-FN and/or the ED-A of fibronectin and/or for any other desired property.

Variable domain amino acid sequence variants of any of the VH and VL domains whose sequences are specifically disclosed herein may be employed in accordance with the present invention, as discussed. Particular variants may include one or more amino acid sequence alterations (addition, deletion, substitution and/or insertion of an amino acid residue), may be less than about 20 alterations, less than about 15 alterations, less than about 10 alterations or less than about 5 alterations, maybe 5, 4, 3, 2 or 1. Alterations may be made in one or more framework regions and/or one or more CDRs. The alterations normally do not result in loss of function, so a an antibody comprising a thus-altered amino acid sequence may retain an ability to bind A-FN and/or the ED-A of fibronectin. For example, it may retain the same quantitative binding as an antibody member in which the alteration is not made, e.g. as measured in an assay described herein. The antibody comprising a thus-altered amino acid sequence may have an improved ability to bind A-FN and/or the ED-A of fibronectin.

Novel VH or VL regions carrying CDR-derived sequences for use in the invention may be generated using random mutagenesis of one or more selected VH and/or VL genes to generate mutations within the entire variable domain. For example, one or two amino acid substitutions are made within an entire variable domain or set of CDRs. Another method that may be used is to direct mutagenesis to CDR regions of VH or VL genes.

As noted above, a CDR amino acid sequence substantially as set out herein may be carried as a CDR in a human antibody variable domain or a substantial portion thereof. The HCDR3 sequences substantially as set out herein represent embodiments of the present invention and for example each of these may be carried as a HCDR3 in a human heavy chain variable domain or a substantial portion thereof.

Variable domains may be obtained or derived from any germ-line or rearranged human variable domain, or may be a synthetic variable domain based on consensus or actual sequences of known human variable domains. A variable domain can be derived from a non-human antibody. A CDR sequence (e.g. CDR3) may be introduced into a repertoire of variable domains lacking a CDR (e.g. CDR3), using recombinant DNA technology. For example, Marks et al. (1992) describe methods of producing repertoires of antibody variable domains in which consensus primers directed at or adjacent to the 5' end of the variable domain area are used in conjunction with consensus primers to the third framework region of human VH genes to provide a repertoire of VH variable domains lacking a CDR3. Marks et al. further describe how this repertoire may be combined with a CDR3 of a particular antibody. Using analogous techniques, the CDR3-derived sequences of the present invention may be shuffled with repertoires of VH or VL domains lacking a CDR3, and the shuffled complete VH or VL domains combined with a cognate VL or VH domain to provide antibodies for use in the invention. The repertoire may then be displayed in a suitable host system such as the phage display system of WO92/01047, or any of a subsequent large body of literature, including Kay, Winter & McCafferty (1996), so that suitable antibodies may be selected. A repertoire may consist of from anything from 10⁴ individual members upwards, for example at least 10⁵, at least 10⁶, at least 10⁷, at least 10⁸, at least 10⁹ or at least 10¹⁰ members.

Similarly, one or more, or all three CDRs may be grafted into a repertoire of VH or VL domains that are then screened for an antibody or antibodies for the A-FN and/or the ED-A of fibronectin.

One or more of the HCDR1, HCDR2 and HCDR3 of antibody H1, B2, C5, D5, E5, C8, F8, F1, B7, E8 or G9 , or the set of HCDRs may be employed, and/or one or more of the LCDR1, LCDR2 and LCDR3 of antibody H1, B2, C5, D5, E5, C8, F8, F1, B7, E8 or G9 or the set of LCDRs of antibody H1, B2, C5, D5, E5, C8, F8, F1, B7, E8 or G9 may be employed.

Similarly, other VH and VL domains, sets of CDRs and sets of HCDRs and/or sets of LCDRs disclosed herein may be employed.

The A-FN and/or the ED-A of fibronectin may also be used in a screen for antibody molecules for use in the preparation of a medicament for the treatment of a lymphoma. The screen may a screen of a repertoire as disclosed elsewhere herein.

A substantial portion of an immunoglobulin variable domain may comprise at least the three CDR regions, together with their intervening framework regions. The portion may also include at least about 50% of either or both of the first and fourth framework regions, the 50% being the C-terminal 50% of the first framework region and the N-terminal 50% of the fourth framework region. Additional residues at the N-terminal or C-terminal end of the substantial part of the variable domain may be those not normally associated with naturally occurring variable domain regions. For example, construction of antibodies made by recombinant DNA techniques may result in the introduction of N- or C-terminal residues encoded by linkers introduced to facilitate cloning or other manipulation steps. Other manipulation steps include the introduction of linkers to join variable domains disclosed elsewhere herein to further protein sequences including antibody constant regions, other variable domains (for example in the production of diabodies) or detectable/functional labels as discussed in more detail elsewhere herein.

Although antibodies may comprise a pair of VH and VL domains, single binding domains based on either VH or VL domain sequences may also be used in the invention. It is known that single immunoglobulin domains, especially VH domains, are capable of binding target antigens in a specific manner. For example, see the discussion of dAbs above.

In the case of either of the single binding domains, these domains may be used to screen for complementary domains capable of forming a two-domain antibody able to bind A-FN and/or the ED-A of fibronectin. This may be achieved by phage display screening methods using the so-called hierarchical dual combinatorial approach as disclosed in WO92/01047, in which an individual colony containing either an H or L chain clone is used to infect a complete library of clones encoding the other chain (L or H) and the resulting two-chain antibody is selected in accordance with phage display techniques such as those described in that reference. This technique is also disclosed in Marks 1992.

Antibodies for use in the present invention may further comprise antibody constant regions or parts thereof, e.g. human antibody constant regions or parts thereof. For example, a VL domain may be attached at its C-terminal end to antibody light chain constant domains including human Cκ or Cλ chains, e.g. Cλ. Similarly, an antibody based on a VH domain may be attached at its C-terminal end to all or part (e.g. a CHl domain) of an immunoglobulin heavy chain derived from any antibody isotype, e.g. IgG, IgA, IgE and IgM and any of the isotype sub-classes, particularly IgG1 and IgG4. Any synthetic or other constant region variant that has these properties and stabilizes variable regions is also useful in embodiments of the present invention.

Antibodies for use in the invention may be labelled with a detectable or functional label. A label can be any molecule that produces or can be induced to produce a signal, including but not limited to fluorescers, radiolabels, enzymes, chemiluminescers or photosensitizers. Thus, binding may be detected and/or measured by detecting fluorescence or luminescence, radioactivity, enzyme activity or light absorbance. Detectable labels may be attached to antibodies for use in the invention using conventional chemistry known in the art.

There are numerous methods by which the label can produce a signal detectable by external means, for example, by visual examination, electromagnetic radiation, heat, and chemical reagents. The label can also be bound to another binding member that binds the antibody for use in the invention, or to a support.

Labelled antibodies, e.g. scFv labelled with a detectable label, may be used diagnostically in vivo, *ex vivo* or *in vitro,* and/or therapeutically.

For example, radiolabelled antibodies (e.g. antibodies conjugated to a radioisotope) may be used in radiodiagnosis and radiotherapy. Radioisotopes which may be conjugated to an antibody for use in the invention include isotopes such as ^{94m}Tc, ^{99m}Tc, ¹⁸⁶Re, ¹⁸⁸Re, ²⁰³Pb, ⁶⁷Ga, ⁶⁸Ga, ⁴⁷Sc, ¹¹¹In, ⁹⁷Ru, ⁶²Cu, ⁶⁴Cu, ⁸⁶Y, ⁸⁸Y, ⁹⁰Y, ¹²¹Sn, ¹⁶¹Tb, ¹⁵³Sm, ¹⁶⁶Ho, ¹⁰⁵Rh, ¹⁷⁷Lu, ¹²³I, ¹²⁴I, ¹²⁵I and ¹³¹I.

For example, an antibody for use in the invention labelled with a detectable label may be used to detect, diagnose or monitor a lymphoma in a human or animal.

An antibody as disclosed herein may be used for the manufacture of a diagnostic product for use in diagnosing a lymphoma.

The invention also provides an antibody for use in an *in vivo* method of diagnosing lymphoma in a human or animal, wherein the method comprises the steps of:
(a) administering to the human or animal an antibody, for example labelled with a detectable label, which binds the ED-A isoform of fibronectin and/or the ED-A of fibronectin, and
(b) determining the presence or absence of the antibody in the lymphatic system of the human or animal body;
wherein localisation of the antibody to the lymphatic system in the human or animal indicates the presence of a lymphoma.

Where the antibody is labelled with a detectable label, the presence or absence of the detectable label may be determined by detecting the label.

A conjugate or fusion between an antibody for use in the invention and a molecule that exerts a biocidal or cytotoxic effect on target cells in the lesions and an antibody directed against an extracellular matrix component which is present in such lesions may be employed in the present invention. For example, the biocidal or cytotoxic molecule may be interleukin-2 (IL-2), doxorubicin, interleukin-12 (IL-12), Interferon-γ (IFN-γ), Tumour Necrosis Factor α (TNFα) or tissue factor (preferably truncated). Such conjugates may be used therapeutically, e.g. for treatment of lymphoma as referred to herein.

Production and use of fusions or conjugates of antibodies with biocidal or cytotoxic molecules is described for example in WO01/62298.

Also disclosed herein is a method of treating lymphoma, the method comprising administering a to an individual a therapeutically effective amount of a medicament comprising a an antibody for use in the invention.

The antibody may be a conjugate of (i) a molecule which exerts a biocidal or cytotoxic effect on target cells by cellular interaction and (ii) an antibody for the ED-A isoform of fibronectin and/or the ED-A of fibronectin.

The invention also provides the use of an antibody for use in the invention for the preparation of a medicament for the treatment of a lymphoma.

The antibody may be a conjugated or fused to a molecule that exerts a biocidal or cytotoxic effect as described herein. The antibody may be a conjugate of (i) a molecule which exerts a biocidal or cytotoxic effect on target cells by cellular interaction and (ii) an antibody for human fibronectin as described herein.

Also described herein is a conjugate of (i) a molecule which exerts a biocidal or cytotoxic effect on target cells by cellular interaction and (ii) an antibody for human fibronectin according for use in the present invention. Such a conjugate preferably comprises a fusion protein comprising the biocidal or cytotoxic molecule and a said antibody , or, where the antibody member is two-chain or multi-chain, a fusion protein comprising the biocidal or cytotoxic molecule and a polypeptide chain component of said antibody. Preferably the antibody is a single-chain polypeptide, e.g. a single-chain antibody molecule, such as scFv. A fusion protein comprising the biocidal or cytotoxic molecule and a single-chain Fv antibody molecule may be used in the invention.

The biocidal or cytotoxic molecule that exerts its effect on target cells by cellular interaction, may interact directly with the target cells, may interact with a membrane-bound receptor on the target cell or perturb the electrochemical potential of the cell membrane. Molecules which interact with a membrane-bound receptor include chemokines, cytokines and hormones. Compounds which perturb the electrochemical potential of the cell membrane include hemolysin, ionophores, drugs acting on ion channels. In exemplary preferred embodiments the molecule is interleukin-2, tissue factor (preferably truncated) or doxorubicin. Other embodiments may employ interleukin 12, interferon-gamma, IP-10 and Tumor Necrosis Factor-α (TNF-α).

As discussed further below, a specific binding member as referred to herein is preferably an antibody. Conveniently, the antibody may be a single-chain polypeptide, such as a single-chain antibody. This allows for convenient production of a fusion protein comprising single-chain antibody and the biocidal or cytotoxic molecule (e.g. interleukin-2 or tissue factor). An antibody antigen-binding site may be provided by means of association of an antibody VH domain and an antibody VL domain in separate polypeptides, e.g. in a complete antibody or in an antibody fragment such as Fab or diabody. Where the antibody is a two-chain or multi-chain molecule (e.g. Fab or whole antibody, respectively), the biocidal or cytotoxic molecule may be conjugated as a fusion polypeptide with one or more polypeptide chains in the antibody.

The antibody may be conjugated with the biocidal or cytotoxic molecule by means of a peptide bond, i.e. within a fusion polypeptide comprising said molecule and the antibody or a polypeptide chain component thereof. See Taniguchi et al. (1983) Nature 302, 305-310; MaED-A et al. (1983) Biochem. Biophys. Res. Comm. 115: 1040-1047; Devos et al. (1983) Nucl. Acids Res. 11: 4307-4323 for IL-2 sequence information useful in preparation of a fusion polypeptide comprising IL-2. Sequence information for truncated tissue factor is provided by Scarpati et al. (1987) Biochemistry 26: 5234-5238, and Ruf et al. (1991) J. Biol. Chem. 226: 15719-15725. Other means for conjugation include chemical conjugation, especially cross-linking using a bifunctional reagent (e.g. employing DOUBLE-REAGENTS^{™} Cross-linking Reagents Selection Guide, Pierce).

Where slow release is desirable, e.g. where the biocidal or cytotoxic molecule is doxorubicin or other molecule which perturbs the electrochemical potential of the cell membrane, chemical conjugation may be by means of formation of a Schiff base (imine) between a primary amino group of the antibody or antibody fragment and an oxidised sugar moiety (daunosamine) of the biocidal or cytotoxic molecule such as doxorubicin.

Also described herein is an isolated nucleic acid encoding a an antibody for use in the present invention. Nucleic acid may include DNA and/or RNA. A nucleic acid may code for a CDR or set of CDRs or VH domain or VL domain or antibody antigen-binding site or antibody molecule, e.g. scFv or IgG, e.g. IgG1, as defined above. The nucleotide sequences may encode the VH and/or VL domains disclosed herein.

Further described herein are constructs in the form of plasmids, vectors, transcription or expression cassettes which comprise at least one polynucleotide as described above.

A recombinant host cell that comprises one or more constructs as above are also described. A nucleic acid encoding any CDR or set of CDRs or VH domain or VL domain or antibody antigen-binding site or antibody molecule, e.g. scFv or IgG1 or IgG4 as provided, is described, as is a method of production of the encoded product, which method comprises expression from encoding nucleic acid. Expression may conveniently be achieved by culturing under appropriate conditions recombinant host cells containing the nucleic acid. Following production by expression a VH or VL domain, or antibody may be isolated and/or purified using any suitable technique, then used as appropriate.

A nucleic acid may comprise DNA or RNA and may be wholly or partially synthetic. Reference to a nucleotide sequence as set out herein encompasses a DNA molecule with the specified sequence, and encompasses a RNA molecule with the specified sequence in which U is substituted for T, unless context requires otherwise.

A method of production of an antibody VH variable domain, the method including causing expression from encoding nucleic acid is also described. Such a method may comprise culturing host cells under conditions for production of said antibody VH variable domain.

A method of production may comprise a step of isolation and/or purification of the product. A method of production may comprise formulating the product into a composition including at least one additional component, such as a pharmaceutically acceptable excipient.

Systems for cloning and expression of a polypeptide in a variety of different host cells are well known. Suitable host cells include bacteria, mammalian cells, plant cells, filamentous fungi, yeast and baculovirus systems and transgenic plants and animals. The expression of antibodies and antibody fragments in prokaryotic cells is well established in the art. For a review, see for example Plückthun 1991. A common bacterial host is *E.coli.*

Expression in eukaryotic cells in culture is also available to those skilled in the art as an option for production of an antibody for example Chadd & Chamow (2001), Andersen & Krummen (2002), Larrick & Thomas (2001). Mammalian cell lines available in the art for expression of a heterologous polypeptide include Chinese hamster ovary (CHO) cells, HeLa cells, baby hamster kidney cells, NSO mouse melanoma cells, YB2/0 rat myeloma cells, human embryonic kidney cells, human embryonic retina cells and many others.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids e.g. phagemid, or viral e.g. 'phage, as appropriate. For further details see, for example, Sambrook & Russell (2001). Many known techniques and protocols for manipulation of nucleic acid, for example in preparation of nucleic acid constructs, mutagenesis, sequencing, introduction of DNA into cells and gene expression, and analysis of proteins, are described in detail in Ausubel 1999.

A host cell may contain a nucleic acid as described herein. Such a host cell may be *in vitro* and may be in culture. Such a host cell may be *in vivo. In vivo* presence of the host cell may allow intracellular expression of an antibody for use in the present invention as "intrabodies" or intracellular antibodies. Intrabodies may be used for gene therapy.

A method comprising introducing a nucleic acid disclosed herein into a host cell is also described. The introduction may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. Introducing nucleic acid in the host cell, in particular a eukaryotic cell may use a viral or a plasmid based system. The plasmid system may be maintained episomally or may incorporated into the host cell or into an artificial chromosome. Incorporation may be either by random or targeted integration of one or more copies at single or multiple loci. For bacterial cells, suitable techniques may include calcium chloride transformation, electroporation and transfection using bacteriophage.

The introduction may be followed by causing or allowing expression from the nucleic acid, e.g. by culturing host cells under conditions for expression of the gene. The purification of the expressed product may be achieved by methods known to one of skill in the art.

The nucleic acid may be integrated into the genome (e.g. chromosome) of the host cell. Integration may be promoted by inclusion of sequences that promote recombination with the genome, in accordance with standard techniques.

A method that comprises using a construct as stated above in an expression system in order to express an antibody or polypeptide as above is also described.

Antibodies for use in the present invention are designed to be used in methods of diagnosis or treatment in human or animal subjects, e.g. human. Antibodies for use in the invention may be used in diagnosis or treatment of lymphoma.

Accordingly, the invention provides use of such a an antibody in the manufacture of a medicament for administration, for example in a method of making a medicament or pharmaceutical composition comprising formulating the antibody member with a pharmaceutically acceptable excipient. Pharmaceutically acceptable vehicles are well known and will be adapted by the person skilled in the art as a function of the nature and of the mode of administration of the active compound(s) chosen.

Antibodies for use in the present invention will usually be administered in the form of a pharmaceutical composition, which may comprise at least one component in addition to the antibody.
Thus pharmaceutical compositions according to the present invention, and for use in accordance with the present invention, may comprise, in addition to active ingredient, a pharmaceutically acceptable excipient, carrier, buffer, stabilizer or other materials well known to those skilled in the art. Such materials should be non-toxic and should not interfere with the efficacy of the active ingredient. The precise nature of the carrier or other material will depend on the route of administration, which may be oral, inhaled or by injection, e.g. intravenous.

Pharmaceutical compositions for oral administration such as for example nanobodies etc are also envisaged in the present invention. Such oral formulations may be in tablet, capsule, powder, liquid or semi-solid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives may be employed, as required. Many methods for the preparation of pharmaceutical formulations are known to those skilled in the art. See, e.g., Robinson, 1978.

A composition may be administered alone or in combination with other treatments, concurrently or sequentially or as a combined preparation with another therapeutic agent or agents, dependent upon the condition to be treated.

An antibody for use in the present invention may be used as part of a combination therapy in conjunction with an additional medicinal component. Combination treatments may be used to provide significant synergistic effects, particularly the combination of a an antibody for use in the present invention with one or more other drugs. An antibody for use in the present invention may be administered concurrently or sequentially or as a combined preparation with another therapeutic agent or agents, for the treatment of one or more of the conditions listed herein.

For example, an antibody for use in the invention may be used in combination with an existing therapeutic agent for the treatment of lymphoma.

Existing therapeutic agents for the treatment of non-Hodgkin's lymphomas include: Rituximab; and Cytoxan, Hydroxyrubicin (Adriamycin), Oncovin (Vincristine), and Prednisone in combination (CHOP chemotherapy regimen).

Existing therapeutic agents for the treatment of Hodgkin's lymphomas include: Adriamycin, bleomycin, vinblastine, and dacarbazine in combination (ABVD chemotherapy regimen).

An antibody for use in the invention and one or more of the above additional medicinal components may be used in the manufacture of a medicament. The medicament may be for separate or combined administration to an individual, and accordingly may comprise the antibody and the additional component as a combined preparation or as separate preparations. Separate preparations may be used to facilitate separate and sequential or simultaneous administration, and allow administration of the components by different routes e.g. oral and parenteral administration.

In accordance with the present invention, compositions provided may be administered to mammals. Administration may be in a "therapeutically effective amount", this being sufficient to show benefit to a patient. Such benefit may be at least amelioration of at least one symptom. The actual amount administered, and rate and time-course of administration, will depend on the nature and severity of what is being treated, the particular mammal being treated, the clinical condition of the individual patient, the cause of the disorder, the site of delivery of the composition, the type of antibody , the method of administration, the scheduling of administration and other factors known to medical practitioners. Prescription of treatment, e.g. decisions on dosage etc, is within the responsibility of general practitioners and other medical doctors, and may depend on the severity of the symptoms and/or progression of a disease being treated. Appropriate doses of antibody are well known in the art (Ledermann 1991 and Bagshawe 1991. Specific dosages indicated herein, or in the Physician's Desk Reference (2003) as appropriate for the type of medicament being administered, may be used. A therapeutically effective amount or suitable dose of an antibody for use in the invention can be determined by comparing its *in vitro* activity and *in vivo* activity in an animal model. Methods for extrapolation of effective dosages in mice and other test animals to humans are known. The precise dose will depend upon a number of factors, including whether the antibody is for diagnosis, prevention or for treatment, the size and location of the area to be treated, the precise nature of the antibody (e.g. whole antibody, fragment or diabody), and the nature of any detectable label or other molecule attached to the antibody. A typical antibody dose will be in the range 100 µg to 1 g for systemic applications, and 1 µg to 1 mg for topical applications. An initial higher loading dose, followed by one or more lower doses, may be administered. An antibody may be a whole antibody, e.g. the IgG1 or IgG4 isotype. This is a dose for a single treatment of an adult patient, which may be proportionally adjusted for children and infants, and also adjusted for other antibody formats in proportion to molecular weight. Treatments may be repeated at daily, twice-weekly, weekly or monthly intervals, at the discretion of the physician. Treatments may be every two to four weeks for subcutaneous administration and every four to eight weeks for intravenous administration. In some embodiments of the present invention, treatment is periodic, and the period between administrations is about two weeks or more, e.g. about three weeks or more, about four weeks or more, or about once a month. In other embodiments of the invention, treatment may be given before, and/or after surgery, and may be administered or applied directly at the anatomical site of surgical treatment.

Further aspects and embodiments of the invention will be apparent to those skilled in the art given the present disclosure including the following experimental exemplification.

### EXPERIMENTAL

### MATERIALS AND METHODS

### Antibodies

The isolation of the anti-ED-B antibody fragment scFv(L19) has been previously described (Pini et al. 1998). The parent anti-ED-A antibody was isolated from the ETH-2 library using published procedures (Giovannoni, Nucleic. Acid Research, 2001, 29(5):E27). The affinity maturation of the parent anti-ED-A antibody, yielding the high affinity anti-ED-A antibodies, is described in the following section.

### Affinity maturation of the parent anti-ED-A antibody

The parent anti-ED-A antibody (an ETH-2-derived antibody) was used as template for the construction of an affinity maturation library. Sequence variability in the VH CDR1 (DP47 germline) and VL CDR1 (DPK22 germline) of the library was introduced by PCR using partially degenerate primers 5'-CTGGAGCCTGGCGGACCCAGCTCATMNNMNNMNNGCTAAAGGTGAAT CCAGA-3¹ (SEQ ID NO: 17) for VH and 5'-CCAGGTTTCTGCTGGTACCAGGCTAA MNNMNNMNNGCTAACACTCTGACTGGCCCTGC-3' (SEQ ID NO: 18) for VL (all oligonucleotides were purchased from Operon Biotechnologies, Cologne, Germany), in a process that generates random mutations at positions 31, 32 and 33 of the VH CDR1 and at positions 31, 31a and 32 of the VL CDR1. VHVL combinations were assembled in scFv format by PCR assembly using the primers LMB3long (5'-CAGGAAACAGCTATGACCATGATTAC-3') (SEQ ID NO: 19) and fdseqlong (5'-GACGTTAGTAAATGAATTTTCTGTATGAGG-3') (SEQ ID NO: 20), using gel-purified VH and VL segments as templates. The assembled VH-VL fragments were doubly digested with NcoI/NotI and cloned into NcoI/NotI-digested pHEN1 phagemid vector (Hoogenboom et al., 1991). The resulting ligation product was electroporated into electrocompetent E. *coli* TG-1 cells according to (Viti et al., 2000), giving rise to a library containing 1.5 x 10⁷ individual antibody clones, which was screened for antibodies which bind ED-A with improved affinity.

### Selection of anti-ED-A antibodies

The antibody library described above was screened for antibodies which bound ED-A with a greater affinity than the parent anti-ED-A antibody using BIAcore analysis. The antigen (11A12) used in the BIAcore analysis contained the ED-A domain of human fibronectin and has the following amino acid sequence (SEQ ID NO: 120):

The nucleotide sequence of antigen (11A12) (SEQ ID NO: 121) is as follows:

The nucleotide sequence of the antigen was amplified by PCR using primers containing BamHI and BglII restriction sites at the 5' and 3' respectively. The resulting PCR product and the vector pQE12 (QIAGEN) were digested with BamHI and BglII restriction endonuclease and subsequently ligated in a reaction containing a ratio of insert to vector of 3:1. The resulting vector was sequenced to check that the sequence was correct.

The antigen was prepared as follows:

A TG1 electrocompetent Preculture in 10 ml 2TY, Amp, 1% Glucose was electroporated in the presence of 1 µl of a DNA miniprep of 11A12. The pre-culture was then diluted 1:100 (8ml in 800ml of 2TY, Amp, 0.1% Glucose) and grown to an OD600 of 0.4-0.6 and then induced with IPTG over night. The following day the cells were spun down and the supernatant filtered (Millipore 0,22 µm). After centrifugation and clarification of the culture broth, 11A12 was purified using a Hitrap column on FPLC. The Ni/ column was regenerated as follows: the column was rinsed with 5 column volumes (CV) H2O followed by application of 3CV 0.5 M EDTA/0.2 M Tris pH 8 to wash the old Nickel out from the column. This was followed by rinsing of the column with 5CV H2O. The column was then reloaded with 2CV 100 mM NiSO4 followed by rinsing of the column with several CVs H2O. The column was then equilibrated with 5CV lysis buffer (20 mM imidazol /250 mM NaCl/ PBS pH 7.4). The cell lysate was filtered (Millipore 0.45 µm) and loaded onto the column (manually). The column was then put back on FPLC and the lysis buffer left to flow until the UV signal was stable (constant), about 3 CV. The elution program was then started: Gradient from 0% to 100% of Elution Buffer (400 mM imidazol/250 mM NaCl/ PBS pH 7.4) in 5CV. The fractions containing the eluted antigen were pooled and dialysed in PBS over night.

### Expression and Purification of the anti-ED-A antibodies

The anti-ED-A antibodies were expressed and purified as follows: A TGl electrocompetent Preculture in 10 ml 2TY, Amp, 1%Glucose was electroporated in the presence of 1 µl of a DNA miniprep of one of the anti-ED-A antibodies. The pre-culture was then diluted 1:100 (8ml in 800ml of 2TY, Amp, 0.1%Glucose) and grown to an OD600 of 0.4-0.6 and then induced with IPTG over night. The following day the cells were spun down and the supernatant filtered (Millipore 0,22 µm). The scFv were purified on a Protein A-Sepharose column and Triethylemmine was used to elute the scFvs from the column. The fractions containing the eluted scFvs were dialysed in PBS over night at 4°C. The scFv fractions were then put on a Superdex 75 column with PBS flowing at 0.5 ml/min and 0.25 ml fractions collected. The monomeric fractions were used for BIAcore analysis.

### BIAcore analysis 1

The BIAcore Chip was flushed overnight at a flow rate of 5 µl/min with HBS-EP buffer BIACORE, 0.01 M Hepes pH 7.4, 0.15 M NaCl, 3 mM EDTA, 0.005% surfactant P20 (same buffer used for the assay). The antigen (11A12) was diluted to a concentration of 50 µg/ml in acetate buffer (pH 4.0) and the COOH groups on the chip were activated by injection of 50 µl of a mix of N-Hydroxy Succinimmide (NHS) and ethyl-N-(dimethylaminopropyl)-carbodiimide (EDC). 40 µl of the 11A12 antigen were injected onto the chip and the residual free COOH groups were blocked with 30 µl of ethanolamine. After a 0,22 µm filtration, 20 µl of each individual bacterial supernatant were injected onto the chip and interaction with the antigen was monitored in real time.

### BIAcore analysis 2

The kₒₙ, k_{off} and K_{D} of the parent anti-ED-A antibody and anti-ED-A antibodies B2, C5, D5, C8, F8, B7 and G9 were evaluated using Surface Plasmon Resonance. The chip was equilibrated over night with the same buffer used during the assay at a buffer flow rate of 5 µl/min. The whole coating procedure was performed at this flow rate. The antigen 11A12 was diluted 1:25 with acetate buffer pH 4.00 (provided by BIACORE) to a final concentration of 20 µg/ml. The NHS and EDC were then mixed and 50µl injected to activate the COOH groups on the CM5 chip. This was followed by injection of 40 µl of the antigen (this lasts about 40"). Then 30 µl of Ethanolammine were injected in order to block the reactivity of eventual free COOH.

Each sample was assayed at a flow rate 20 µl/min. 20 µl of undiluted monomeric protein (as it comes out from the gel filtration) was injected. The dissociation time was left to run for about 200". Then 10 µl of HCl 10mM was injected to regenerate the chip. The injection of monomeric protein was repeated at different dilutions, i.e. 1:2 dilution (in PBS) followed by regeneration with HCl. This was followed by a third injection of the protein, at a dilution of 1:4 followed again by regenartion with HCl. The kₒₙ, k_{off} and KD values for each anti-ED-A antibody were evaluated using the BIAevaluation software.

### Immunohistochemistry of lymphoma sections

Sections of Ramos lymphoma were fixed in cold acetone (-20°C) for 10 minutes and the slides left to dry at room temperature (RT) for 30 minutes. The slides were then immersed in TBS for 5 to 10 minutes and the back of the slides dried with paper without touching the sections. This was followed by blocking of the sections with >100 µl of 20% Foetal Calf Serum (FCS) in TBS (50 mM TRIS, 100 mM NaCl, adjusted to pH 7.4, 0.01% Aprotinin) for 30 minutes. The blocking solution was poured off and the slides submerged in TBS for 5 minutes. 100 µl of primary antibody scFv F8 (~20 ng/µl) carrying a myc-tag, together with 10 µl of biotinylated anti-myc antibody 9E10 (OD 0.25, diluted 1:20) diluted in TBS/3% BSA, were then added to the slides. As a negative control, a Ramos lymphoma section was immunohistochemically stained in the same way but omitting the primary antibody, i.e. the myc-tagged scFv anti-ED-A antibody F8. The slides were incubated in a moist chamber for 1 hour. The slides were washed with TBS followed by the addition of Streptavidin-Alkaline Phosphatase diluted 1:150 in TBS/3% BSA and incubation in a moist chamber for 30 minutes. The slides were then washed with TBS twice for 5 minutes and the back of the slides dried with paper. 500 µl of the Fast Red substrate (5 mg FastRed powder added to 5 ml Fast Red solution [49 ml TRIS-HCl, 0.1M, pH 8.2; 1.0 ml N,N-dimethylformammide; 10 mg Naphthol AS-MX Phosphate and 50 µl Levamisole solution (1 ml 0.1 M TRIS-HCl pH 8.2, 240.8 mg Levamisole powder)] and filtered with a filter with a pore size of 0.45 µ was added to each slide and the slides incubated in a moist chamber for 15 minutes. The slides were washed twice with deionised water by directly applying the deionised water onto each section with a plastic Pasteur pipette and then left in water. The slides were then transferred to a Gillis Hematoxilin solution for 50 min followed by a quick transfer to water and rinsed with water 6 times. Finally, the slides were mounted with Glycergel (DakoCytomation, Glostrup, Denmark) mounting medium and analyzed with an Axiovert S100 TV microscope (Carl Zeiss, Feldbach, Switzerland) using the Axiovision software (Carl Zeiss).

### Immunohistochemistry staining of lung cancer sections

Sections of a small cell lung cancer (small cell carcinoma) and of several non-small cell lung cancers (squamous cell carcinoma, adeno-carcinoma, bronchio-aleveolar carcinoma and large cell carcinoma) were immunohistochemically stained with an scFv anti-ED-A antibody carrying a myc-tag, as previously described (see e.g., Brack et al. 2006). In brief, the sections were incubated with the scFv anti-EDA antibody D5 (final concentration, 2-15 µg/mL) and with the secondary antibody (monoclonal anti-myc antibody 9E10) simultaneously. Bound antibodies were detected with rabbit antimouse immunoglobulin antibody (Dakocytomation, Glostrup, Denmark) followed by mouse monoclonal alkaline phosphatase-anti-alkaline phosphatase complex (Dakocytomation). Fast Red (Sigma) was used as phosphatase substrate, and the sections were counterstained with hematoxylin (Sigma). Finally, the sections were mounted with Glycergel (DakoCytomation, Glostrup, Denmark) and analyzed with an Axiovert S100 TV microscope (Carl Zeiss, Feldbach, Switzerland) using the Axiovision software (Carl Zeiss).

### RESULTS

### Selection of anti-ED-A antibodies

### BIAcore analysis 1

The BIAcore analysis produced a graph for each anti-ED-A antibody which was analysed to deduce the affinity of an antibody for the antigen as follows: The x axis of each graph corresponds to time and the y axis corresponds to Resonance Units (a measure which indicates the binding affinity of the tested antibody for the antigen coated onto the BIAcore chip). Each graph showed 3 peaks and 1 dip which correspond to changes of buffer and are therefore irrelevant for the interpretation of the results.

The ascending part of each graph represents the association phase. The steeper the curve in this part of the graph, the faster the association of the antibody with the antigen. The descending part of each graph represents the dissociation phase of the antibody from the antigen. The flatter the curve in this part of the graph is, the slower the dissociation of the antibody from the antigen.

Anti-ED-A antibodies H1, B2, C5, D5, E5, C8, F8, F1, B7, E8 and G9 all showed a flatter dissociation curve than the parent anti-ED-A antibody from which they were derived, indicating that they bind ED-A, and hence also A-FN, with a greater affinity than the parent anti-ED-A antibody. The graphs for antibodies E5, F1, F8 and H1 showed the flattest dissociation curves of all the anti-ED-A antibodies tested. The association curves of antibodies H1, C5, D5, E5, C8, F8 and F1 were flatter than that observed for the parent anti-ED-A antibody while the association curve observed for antibodies B2, B7, E8 and G9 was as steep as the association curve observed for the parent anti-ED-A antibody. However, as bacterial supernatants of IPTG-induced *E. coli* TG-1 cells were used for the BIAcore analysis of antibodies H1, B2, C5, D5, E5, C8, F8, F1, B7, E8 and G9, the concentration of the tested antibody samples was unknown but most probably lower than the concentration of the parent anti-ED-A antibody sample used for comparison. Consequently, the association curve of antibodies H1, B2, C5, D5, E5, C8, F8, F1, B7, E8 and G9 may be artificially low due to the low concentration of antibody in the samples used for the BIAcore analysis. However, as concentration does not significantly affect the dissociation of an antibody from its target antigen in BIAcore analysis, the flat dissociation curves observed for antibodies H1, B2, C5, D5, E5, C8, F8, F1, B7, E8 and G9 show that these antibodies bind ED-A with at least an equal, and probably a higher affinity, than the parent anti-ED-A antibody.

### BIAcore analysis 2

The kₒₙ, k_{off} and KD values for each anti-ED-A antibody were evaluated using the BIAevaluation software. The kₒₙ, k_{off} and KD values of the parent anti-ED-A antibody and anti-ED-A antibodies B2, C5, D5, C8, F8, B7 and G9 for antigen 11A12 are detailed in Table 2. Anti-ED-A antibodies B2, C5, D5, C8, F8, B7 and G9 all have a better K_{D} values for antigen 11A12 than the parent anti-ED-A antibody from which they were derived, indicating that they bind ED-A, and hence also A-FN, with a greater affinity than the parent anti-ED-A antibody.

### Immunohistochemistry of lymphoma sections

Immunohistochemical staining of sections of primary human Ramos lymphoma (a non-Hodgkin's B-cell lymphoma [Burkitt's lymphoma]) with anti-ED-A scFv F8 antibody showed a strong and specific staining of the neovasculature. In contrast no staining of the primary Ramos lymphoma, including the neovasculature, was detected in the negative control in which the primary Ramos lymphoma was stained under identical conditions except for the omission of the anti-ED-A scFv F8 antibody. This demonstrates that the anti-EDA scFv antibodies of the present invention are specifically targeted to the neovasculature of lymphomas. ED-A may therefore serve as a general target for antibody based targeting strategies in lymphoma.

### Immunohistochemistry of lung cancer sections

It is generally difficult to find 'pantumoral antibodies' within a certain class of cancer, for example Herceptin stains only 20% of breast cancers. Figure 1 shows that anti-ED-A antibody F8 specifically localises to the neovasculature of lung cancers. Specifically, anti-ED-A antibody F8 localises specifically to the neovasculature of both small cell lung cancer and non-small cell lung cancer. Non-small cell lung cancers account for ~75%-85% of all lung cancers, while small cell lung cancers account for ~15%-25%. Figure 1 further demonstrates that anti-ED-A antibody specifically localises to all non-small cell lung cancer subtypes tested, namely squamous cell carcinoma, adeno-carcinoma, bronchio-alveolar carcinoma and large cell carcinoma. Thus the results shown in Figure 1 surprisingly demonstrate that anti-ED-A antibody, F8, stains all histotypes of lung cancer tested. ED-A may therefore serve as a general target for antibody based targeting strategies in lung cancer.

### Sequencing

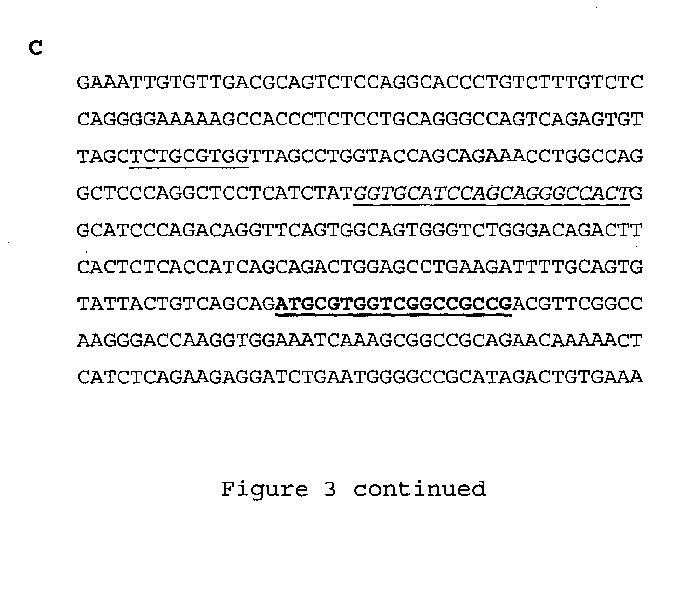

Anti-ED-A antibodies H1, B2, C5, D5, E5, C8, F8, F1, B7, E8 and G9 are all scFv antibodies and were sequenced using conventional methods. The nucleotide sequence of the anti-ED-A antibody H1 is shown in Figure 3. The amino acid sequence of the anti-ED-A antibody H1 is shown in Figure 4.

Preferred nucleotide sequences encoding VH and/or VL of anti-ED-A antibodies B2, C5, D5, E5, C8, F8, F1, B7, E8 and G9 are identical to nucleotide sequences encoding VH and/or VL of anti-ED-A antibody H1, except that the nucleotide sequences encoding the H1 CDR1s of the light (VL) and heavy (VH) chain are substituted with the nucleotide sequences encoding the light (VL) and heavy (VH) chain CDR1s listed in Table 1 for the respective antibody.

The preferred nucleotide sequences encoding the VH and/or VL of anti-ED-A scFv F8 diabody are identical to the nucleotide sequences encoding VH and/or VL of anti-ED-A antibody H1, except that the nucleotide sequences encoding the H1 CDR1s of the light (VL) and heavy (VH) chain are substituted with the nucleotide sequences encoding the light (VL) and heavy (VH) chain CDR1s listed in Table 1 for anti-ED-A antibody F8. The preferred nucleotide sequence encoding the linker linking the VH and VL of the anti-ED-A scFv F8 diabody is gggtccagtggcggt (SEQ ID NO: 29).

Anti-ED-A antibodies B2, C5, D5, E5, C8, F8, F1, B7, E8 and G9 have identical amino acid sequences to anti-ED-A antibody H1 except that the amino acid sequences of the H1 CDR1s of the light (VL) and heavy (VH) chain are substituted with the amino acid sequences of the light (VL) and heavy (VH) chain CDR1s listed in Table 1 for the respective antibody. The amino acid sequence of the anti-ED-A scFv F8 diabody is identical to the amino acid sequences of anti-ED-A antibody H1, except that the amino acid sequences of the H1 CDR1s of the light (VL) and heavy (VH) chain are substituted with the amino acid sequences of the light (VL) and heavy (VH) chain CDR1s listed in Table 1 for anti-ED-A antibody F8, and the amino acid sequence of the linker in H1 is substituted with the linker amino acid sequence GSSGG (SEQ ID NO: 28).

The amino acid sequence of the anti-ED-A antibody B2 VH domain (SEQ ID NO: 21) is identical to the amino acid sequence of the VH domain of anti-ED-A antibody H1 except that SEQ ID NO: 23 is substituted for the VH CDR1 of H1.

The amino acid sequence of the anti-ED-A antibody C5 VH domain (SEQ ID NO: 41) is identical to the amino acid sequence of the VH domain of anti-ED-A antibody H1 except that SEQ ID NO: 43 is substituted for the VH CDR1 of H1.

The amino acid sequence of the anti-ED-A antibody D5 VH domain (SEQ ID NO: 51) is identical to the amino acid sequence of the VH domain of anti-ED-A antibody H1 except that SEQ ID NO: 53 is substituted for the VH CDR1 of H1.

The amino acid sequence of the anti-ED-A antibody E5 VH domain (SEQ ID NO: 61) is identical to the amino acid sequence of the VH domain of anti-ED-A antibody H1 except that SEQ ID NO: 63 is substituted for the VH CDR1 of H1.

The amino acid sequence of the anti-ED-A antibody C8 VH domain (SEQ ID NO: 71) is identical to the amino acid sequence of the VH domain of anti-ED-A antibody H1 except that SEQ ID NO: 73 is substituted for the VH CDR1 of H1.

The amino acid sequence of the anti-ED-A antibody F8 VH domain (SEQ ID NO: 81) is identical to the amino acid sequence of the VH domain of anti-ED-A antibody H1 except that SEQ ID NO: 83 is substituted for the VH CDR1 of H1. The VH domain of the anti-ED-A F8 diabody has the same amino acid sequence as VH domain of the anti-ED-A antibody F8 (i.e. SEQ ID NO: 81).

The amino acid sequence of the anti-ED-A antibody F1 VH domain (SEQ ID NO: 91) is identical to the amino acid sequence of the VH domain of anti-ED-A antibody H1 except that SEQ ID NO: 93 is substituted for the VH CDR1 of H1.

The amino acid sequence of the anti-ED-A antibody B7 VH domain (SEQ ID NO: 101) is identical to the amino acid sequence of the VH domain of anti-ED-A antibody H1 except that SEQ ID NO: 103 is substituted for the VH CDR1 of H1.

The amino acid sequence of the anti-ED-A antibody E8 VH domain (SEQ ID NO: 111) is identical to the amino acid sequence of the VH domain of anti-ED-A antibody H1 except that SEQ ID NO: 113 is substituted for the VH CDR1 of H1.

The amino acid sequence of the anti-ED-A antibody G9 VH domain (SEQ ID NO: 31) is identical to the amino acid sequence of the VH domain of anti-ED-A antibody H1 except that SEQ ID NO: 33 is substituted for the VH CDR1 of H1.

The amino acid sequence of the anti-ED-A antibody B2 VL domain (SEQ ID NO: 22) is identical to the amino acid sequence of the VL domain of anti-ED-A antibody H1 except that SEQ ID NO: 26 is substituted for the VL CDR1 of H1.

The amino acid sequence of the anti-ED-A antibody C5 VL domain (SEQ ID NO: 42) is identical to the amino acid sequence of the VL domain of anti-ED-A antibody H1 except that SEQ ID NO: 46 is substituted for the VL CDR1 of H1.

The amino acid sequence of the anti-ED-A antibody D5 VL domain (SEQ ID NO: 52) is identical to the amino acid sequence of the VL domain of anti-ED-A antibody H1 except that SEQ ID NO: 56 is substituted for the VL CDR1 of H1.

The amino acid sequence of the anti-ED-A antibody E5 VL domain (SEQ ID NO: 62) is identical to the amino acid sequence of the VL domain of anti-ED-A antibody H1 except that SEQ ID NO: 66 is substituted for the VL CDR1 of H1.

The amino acid sequence of the anti-ED-A antibody C8 VL domain (SEQ ID NO: 72) is identical to the amino acid sequence of the VL domain of anti-ED-A antibody H1 except that SEQ ID NO: 76 is substituted for the VL CDR1 of H1.

The amino acid sequence of the anti-ED-A antibody F8 VL domain (SEQ ID NO: 82) is identical to the amino acid sequence of the VL domain of anti-ED-A antibody H1 except that SEQ ID NO: 86 is substituted for the VL CDR1 of H1. The VL domain of the anti-ED-A F8 diabody has the same amino acid sequence as VL domain of the anti-ED-A antibody F8 (i.e. SEQ ID NO: 82).

The amino acid sequence of the anti-ED-A antibody F1 VL domain (SEQ ID NO: 92) is identical to the amino acid sequence of the VL domain of anti-ED-A antibody H1 except that SEQ ID NO: 96 is substituted for the VL CDR1 of H1.

The amino acid sequence of the anti-ED-A antibody B7 VL domain (SEQ ID NO: 102) is identical to the amino acid sequence of the VL domain of anti-ED-A antibody H1 except that SEQ ID NO: 106 is substituted for the VL CDR1 of H1.

The amino acid sequence of the anti-ED-A antibody E8 VL domain (SEQ ID NO: 112) is identical to the amino acid sequence of the VL domain of anti-ED-A antibody H1 except that SEQ ID NO: 116 is substituted for the VL CDR1 of H1.

The amino acid sequence of the anti-ED-A antibody G9 VL domain (SEQ ID NO: 32) is identical to the amino acid sequence of the VL domain of anti-ED-A antibody H1 except that SEQ ID NO: 36 is substituted for the VL CDR1 of H1.

Optionally, the amino acid at position 5 of the VH domain of anti-ED-A antibodies H1, B2, C5, D5, E5, C8, F8, F1, B7, E8, G9 and the scFv F8 diabody may be a leucine residue (L) rather than a valine residue (V) as shown in Figure 4A. In addition, or alternatively, the amino acid at position 18 of the VL domain of anti-ED-A antibodies H1, B2, C5, D5, E5, C8, F8, F1, B7, E8, G9 and the scFv F8 diabody may be an arginine residue (R) rather than a lysine residue (K) as shown in Figure 4C.

### REFERENCES

Amit et al. (1986), Science, 233:747-753.
Andersen et al. (2002) Current Opinion in Biotechnology 13: 117
Ausubel et al. (1999) 4^{th} eds., *Short Protocols in Molecular Biology:* A *Compendium of Methods from Current Protocols in Molecular Biology,* John Wiley & Sons.
Bagshawe K.D. et al. (1991) Antibody, Immunoconjugates and Radiopharmaceuticals 4: 915-922
Balza et al. (1988), FEBS Lett., 228: 42-44.
Birchler et al. (1999), J. Immunol. Methods, 231, 239-248.
Bird et al. (1988) Science, 242, 423-426
Borsi et al. (1987), J. Cell. Biol., 104, 595-600.
Borsi et al. (1990), FEBS Lett., 261: 175-178.
Borsi et al. (1995), J. Biol.Chem., 270: 6243-6245.
Borsi et al. (1998), Exp. Cell Res., 240, 244-251.
Brack et al. (2006), Clin. Cancer Res., 12, 3200-3208.
Carnemolla et al. (1989), J. Cell. Biol., 108: 1139-1148.
Caton et al. (1990), J. Immunol., 144:1965-1968.
Chadd et al. (2001), Current Opinion in Biotechnology 12: 188-194
Chothia et al. (1987), J. Mol. Biol., 196:901-917.
Chothia et al. (1989), Nature, 342:877- 883.
Devos et al. (1983), Nucl. Acids Res. 11: 4307-4323.
ffrench-Constant (1995), Exp. Cell Res., 221, 261-271.
Giovannoni, Nucleic. Acid Research, 2001, 29(5):E27.
Glennie M J et al., 1987 J. Immunol. 139, 2367-2375
Haan et al. (2004), BioCentury, 12(5): A1-A6.
Hanahan et al. (2000), Cell 100, 57-70.
Harlow and Lane, Antibodies: A Laboratory Manual, Cold Spring Harbor
Kornblihtt et al. (1984), Nucleic Acids Res. 12, 5853-5868.
Laboratory, Cold Spring Harbor N.Y., pp. 726, 1988
Heikinheimo et al. (1991), Virchows Arch. B Cell Pathol. Incl. Mol. Pathol., 61, 101-109.
Holliger and Bohlen 1999 Cancer and metastasis rev. 18: 411-419.
Holliger et al. (1993a), Proc. Natl. Acad. Sci. USA 90 6444-6448.
Holliger et al. (1993b), Current Opinion Biotechnol 4, 446-449.
Holt et al. (2003) Trends in Biotechnology 21, 484-490.
Hoogenboom et al. (1991), Nucleic Acids Res., 19 (15) 4133-7.
Hu et al. (1996), Cancer Res., 56, 3055-3061.
Huston et al. (1988) PNAS USA, 85, 5879-5883.
Hynes, R.O. (1990). Fibronectins (New York: Springer-Verlag).
Jacobs et al. (2002), Hum. Pathol., 33, 29-38.
Kabat et al. (1987) Sequences of Proteins of Immunological Interest. 4th Edition. US Department of Health and Human Services.
Kabat et al. (1991a), Sequences of Proteins of Immunological Interest, 5th Edition. US Department of Health and Human Services, Public Service, NIH, Washington. (a)
Kabat et al. (1991b), J. Immunol., 147:1709-1719.
Kaspar et al. (2006), Int. J. Cancer, 118, 1331-1339.
Knappik et al., (2000) J. Mol. Biol. 296, 57-86.
Kohler and Milstein, Nature, 256:495-497, 1975
Koide et al. (1998), Journal of Molecular Biology, 284: 1141-1151.
Kontermann et al. (2001), S, Antibody Engineering, Springer-Verlag New York, LLC; ISBN: 3540413545.
Koukoulis et al. (1993), J. Submicrosc. Cytol. Pathol., 25, 285-295.
Koukoulis et al. (1995), Ultrastruct. Pathol., 19, 37-43.
Krebs et al. (2001), Journal of Immunological Methods, 254 67-84.
Larrick JW and Thomas DW (2001) Current Opinion in Biotechnology 12:411-418.
Ledermann J.A. et al. (1991) Int. J. Cancer 47: 659-664
Lohi et al. (1995), Int. J. Cancer, 63, 442-449.
MaED-A et al. (1983) Biochem. Biophys. Res. Comm. 115: 1040-1047;
Matsumoto et al. (1999), Jpn. J. Cancer Res., 90, 320-325.
McCafferty et al., (1990) Nature, 348, 552-554.
Mendez, M. et al., (1997) Nature Genet, 15(2): 146-156.
Merchand et al., 1998 Nature Biotech. 16:677-681
Neri, D., and Bicknell, R. (2005), Nat Rev Cancer 5, 436-446.
Nygren et al. (1997), Current Opinion in Structural Biology, 7: 463-469.
Oyama et al. (1989), J. Biol. Chem., 264, 10331-10334.
Paolella et al. (1988), Nucleic Acids Res. 16, 3545-3557.
Pini et al. (1998), J. Biol. Chem., 273, 21769-21776.
Pluckthun (1991), Bio/Technology 9: 545-551.
Reiter et al. (1996), Nature Biotech, 14, 1239-1245.
Repp et al., 1995 J. Hemat. 377-382.
Ridgeway et al. (1996), Protein Eng., 9, 616-621.
Robinson ed., Sustained and Controlled Release Drug Delivery Systems, Marcel Dekker, Inc., New York, 1978
Roesli et al. (2006), Nature Protocols, 1, 192-199.
Ruf et al. (1991) J. Biol. Chem. 226: 15719-15725.
Rybak et al. (2005), Nat. Methods, 2, 291-298.
Rybak et al. (2006), ChemMedChem., 2, 22-40.
Sambrook and Russell, Molecular Cloning: a Laboratory Manual: 3rd edition, 2001, Cold Spring Harbor Laboratory Press
Scarpati et al. (1987) Biochemistry 26: 5234-5238.
Scarpino et al. (1999) J. Pathol. 188, 163-167.
Scheurer et al. (2005), Proteomics 5, 3035-3039.
Segal et al. (1974), PNAS, 71:4298-4302.
Sharon et al. (1990a), PNAS, 87:4814-4817.
Sharon et al. (1990b), J. Immunol., 144:4863-4869.
Silacci et al. (2003), Proteomics, 5, 2340-2350.
Staerz U. D. and Bevan M. J. 1986 PNAS 83
Suresh et al., 1986 Method Enzymol. 121: 210-228
Taniguchi et al. (1983) Nature 302, 305-310;
Tavian et al. (1994), Int. J. Cancer, 56, 820-825.
Terrana et al. (1987), Cancer Res. 47, 3791-3797.
Thorpe (2004), Clin. Cancer Res., 10, 415-427.
Trachsel et al. (2006), Adv. Drug Deliv. Rev., 58, 735-754.
Viti et al. (2000), Methods Enzymol., 326, 480-505.
Ward et al. (1989), Nature 341, 544-546.
Wess In: BioCentury, The Bernstein Report on BioBusiness, 12(42), A1-A7, 2004

**Table 1**

| Nucleotide and amino acid sequences of the heavy chain (VH) and light chain (VL) CDR1s of the anti-ED-A affinity matured antibodies | | |
|---|---|---|
| **Antibody** | **CDR1 (VH)** | **CDR1 (VL)** |
| **H1** | | |
| **B2** | | |
| **C5** | | |
| **D5** | | |
| **E5** | | |
| **C8** | | |
| **F8** | | |
| **F1** | | |
| **B7** | | |
| **E8** | | |
| **G9** | | |

**Table 2**

| **BIAcore evaluation data** | | | |
|---|---|---|---|
| **Antibody** | **kₒₙ (1/Ms)** | **k_{off} (1/s)** | **K_{D} (M)** |
| **Parent anti-ED-A antibody** | 2.5 × 10⁵ | 0.02 | ~1 × 10⁻⁷ |
| **B2** | 3.8 × 10⁵ | 7.54 × 10⁻³ | ~2 × 10⁻⁸ |
| **C5** | 3.04 × 10⁵ | 9.23 × 10⁻³ | ~3 × 10⁻⁸ |
| **D5** | 4.53 × 10⁵ | 7.6 × 10⁻³ | ~1.7 × 10⁻⁸ |
| **C8** | 3.8 × 10⁵ | 5.3 × 10⁻³ | ~1.4 × 10⁻⁸ |
| **F8** | 4.65 × 10⁵ | 1.4 × 10⁻³ | ~3.1 × 10⁻⁹ |
| **B7** | 2.67 × 10⁵ | 4.5 × 10⁻³ | ~1.68 × 10⁻⁸ |
| **G9** | 3.6 × 10⁵ | 7.54 × 10⁻³ | ~2.09 × 10⁻⁸ |

## Claims

1. An antibody that binds the Extra Domain-A (ED-A) isoform of fibronectin for use in a method of treating lymphoma, wherein the antibody is conjugated to a molecule that has biocidal or cytotoxic activity, or to a radioisotope.

2. An antibody that binds the ED-A isoform of fibronectin for use in an *in vivo* method of detecting or diagnosing lymphoma in a human or animal, wherein the antibody is labelled with a detectable label.

3. An antibody for use according to claim 2, wherein the method of detecting or diagnosing a lymphoma in a human or animal comprises the steps of:
(a) administering to the human or animal an antibody which binds the ED-A isoform of fibronectin, and
(b) determining the presence or absence of the antibody in the lymphatic system of the human or animal body;
wherein localisation of the antibody to the lymphatic system in the human or animal indicates the presence of a lymphoma.

4. An antibody that binds the ED-A isoform of fibronectin for use in a method of delivering a molecule conjugated to the antibody to the neovasculature of a lymphoma in a human or animal, wherein molecule has biocidal or cytotoxic activity, is a detectable label, or is a radioisotope.

5. The use of an antibody that binds the ED-A isoform of fibronectin for the preparation of a medicament for delivery to a lymphoma of a molecule conjugated to the antibody, wherein molecule has biocidal or cytotoxic activity, is a detectable label, or is a radioisotope.

6. An antibody for use according to claim 3, wherein the antibody is labelled with a detectable label.

7. An antibody for use according to any one of claims 1 to 4 or 6, or the use of claim 5, wherein the antibody binds the ED-A of fibronectin.

8. An antibody for use according to any one of claims 1 to 4 or 6 to 7, or the use of claim 5 or 7, wherein the lymphoma is Hodgkin's lymphoma or non-Hodgkin's lymphoma.

9. An antibody for use according to any one of claims 1 to 4 or 6 to 8, or the use claim 5, 7 or 8, wherein the antibody comprises a VH domain and a VL domain, wherein the VH domain comprises a framework and a set of complementarity determining regions HCDR1, HCDR2 and HCDR3, and wherein the VL domain comprising a set of complementarity determining regions LCDR1, LCDR2 and LCDR3 and a framework, wherein:
HCDR1 has amino acid sequence SEQ ID NO: 83,
HCDR2 has amino acid sequence SEQ ID NO: 4,
HCDR3 has amino acid sequence SEQ ID NO: 5;
LCDR1 has amino acid sequence SEQ ID NO: 86,
LCDR2 has amino acid sequence SEQ ID NO: 7, and
LCDR3 has amino acid sequence SEQ ID NO: 8.

10. An antibody for use according to claim 9, or the use of claim 9, wherein the VH domain framework and/or the VL domain framework is a human germline framework.

11. An antibody for use according to claim 9 or 10, or the use of claim 9 or 10, wherein the antibody either comprises the VH domain shown in SEQ ID NO: 81; or comprises the VH domain shown in SEQ ID NO: 81, except that the amino acid at position 5 of the VH domain is a leucine residue (L) rather than a valine residue (V).

12. An antibody for use according to any one of claims 9 to 11, or the use of any one of claims 9 to 11, wherein the antibody either comprises the VL domain shown in SEQ ID NO: 82; or comprises the VL domain shown in SEQ ID NO: 82, except that the amino acid at position 18 of the VL domain is an arginine residue (R) rather than a lysine residue (K).

13. An antibody for use according to any one of claims 1 to 4 or 6 to 12, or the use of any one of claims 5 or 7 to 12, wherein the antibody is a single chain Fv or a diabody.

14. An antibody for use according to any one of claims 1 to 4 or 6 to 13, or the use of any one of claims 5 or 7 to 13, wherein the antibody is a single chain Fv or diabody comprising the VH domain shown in SEQ ID NO: 81, except that the amino acid at position 5 of the VH domain is a leucine residue (L) rather than a valine residue (V), and further comprising the VL domain shown in SEQ ID NO: 82, except that the amino acid at position 18 of the VL domain is an arginine residue (R) rather than a lysine residue (K).

15. An *in vitro* method of diagnosing lymphoma, wherein an antibody that binds the ED-A of fibronectin is used.

## Patentansprüche

1. Antikörper, der die Extradomänen-A- (ED-A-) Isoform von Fibronectin bindet, zur Verwendung in einem Verfahren zur Behandlung eines Lymphoms, worin der Antikörper an ein Molekül, das eine biozide oder zytotoxische Aktivität aufweist, oder an ein Radioisotop konjugiert ist.

2. Antikörper, der die ED-A-Isoform von Fibronectin bindet, zur Verwendung in einem In-vivo-Verfahren zur Detektion oder Diagnose eines Lymphoms in einem Menschen oder Tier, worin der Antikörper mit einer detektierbaren Markierung markiert ist.

3. Antikörper zur Verwendung nach Anspruch 2, worin das Verfahren zur Detektion oder Diagnose eines Lymphoms in einem Menschen oder Tier die folgenden Schritte umfasst:
(a) Verabreichen eines Antikörpers, der die ED-A-Isoform von Fibronectin bindet, an den Menschen oder das Tier und
(b) Bestimmen der Gegenwart oder Abwesenheit des Antikörpers in dem lymphatischen System des menschlichen oder tierischen Körpers;
worin eine Lokalisierung des Antikörpers in dem lymphatischen System des Menschen oder des Tiers auf die Gegenwart eines Lymphoms hinweist.

4. Antikörper, der die ED-A-Isoform von Fibronectin bindet, zur Verwendung in einem Verfahren zur Anlieferung eines Moleküls, das an den Antikörper konjugiert ist, an die neu gebildeten Gefäße eines Lymphoms in einem Menschen oder einem Tier, worin das Molekül eine biozide oder zytotoxische Aktivität aufweist, eine detektierbare Markierung oder ein Radioisotop ist.

5. Verwendung eines Antikörpers, der die ED-A-Isoform von Fibronectin bindet, zur Herstellung eines Medikaments zur Anlieferung eines Moleküls, das an den Antikörper konjugiert ist, an ein Lymphom, worin das Molekül eine biozide oder zytotoxische Aktivität aufweist, eine detektierbare Markierung oder ein Radioisotop ist.

6. Antikörper zur Verwendung nach Anspruch 3, worin der Antikörper mit einer detektierbaren Markierung markiert ist.

7. Antikörper zur Verwendung nach einem der Ansprüche 1 bis 4 oder 6 oder Verwendung nach Anspruch 5, worin der Antikörper die ED-A von Fibronectin bindet.

8. Antikörper zur Verwendung nach einem der Ansprüche 1 bis 4 oder 6 bis 7 oder Verwendung nach Anspruch 5 oder 7, worin das Lymphom ein Hodgkin-Lymphom oder ein Non-Hodgkin-Lymphom ist.

9. Antikörper zur Verwendung nach einem der Ansprüche 1 bis 4 oder 6 bis 8 oder Verwendung nach Anspruch 5, 7 oder 8, worin der Antikörper eine VH-Domäne und eine VL-Domäne umfasst, worin die VH-Domäne ein Gerüst und einen Satz komplementaritätsbestimmender Regionen HCDR1, HCDR2 und HCDR3 umfasst und worin die VL-Domäne einen Satz komplementaritätsbestimmender Regionen LCDR1, LCDR2 und LCDR3 und ein Gerüst umfasst, worin:
HCDR1 Aminosäuresequenz Seq.-ID Nr. 83 aufweist,
HCDR2 Aminosäuresequenz Seq.-ID Nr. 4 aufweist,
HCDR3 Aminosäuresequenz Seq.-ID Nr. 5 aufweist,
LCDR1 Aminosäuresequenz Seq.-ID Nr. 86 aufweist,
LCDR2 Aminosäuresequenz Seq.-ID Nr. 7 aufweist und
LCDR3 Aminosäuresequenz Seq.-ID Nr. 8 aufweist.

10. Antikörper zur Verwendung nach Anspruch 9 oder Verwendung nach Anspruch 9, worin das VH-Domänengerüst und/oder das VL-Domänengerüst ein menschliches Keimbahn-Gerüst ist.

11. Antikörper zur Verwendung nach Anspruch 9 oder 10 oder Verwendung nach Anspruch 9 oder 10, worin der Antikörper entweder die in Seq.-ID Nr. 81 dargelegte VH-Domäne umfasst; oder die in Seq.-ID Nr. 81 dargelegte VH-Domäne umfasst, mit der Ausnahme, dass die Aminosäure an Position 5 der VH-Domäne ein Leucin-Rest (L) anstelle eines Valin-Rests (V) ist.

12. Antikörper zur Verwendung nach einem der Ansprüche 9 bis 11 oder Verwendung nach einem der Ansprüche 9 bis 11, worin der Antikörper entweder die in Seq.-ID Nr. 82 dargelegte VL-Domäne umfasst; oder die in Seq.-ID Nr. 82 dargelegte VL-Domäne umfasst, mit der Ausnahme, dass die Aminosäure an Position 18 der VL-Domäne ein Arginin-Rest (R) anstelle eines Lysin-Rests (K) ist.

13. Antikörper zur Verwendung nach einem der Ansprüche 1 bis 4 oder 6 bis 12 oder Verwendung nach einem der Ansprüche 5 oder 7 bis 12, worin der Antikörper ein Einzelketten-Fv oder ein Diabody ist.

14. Antikörper zur Verwendung nach einem der Ansprüche 1 bis 4 oder 6 bis 13 oder Verwendung nach einem der Ansprüche 5 oder 7 bis 13, worin der Antikörper ein Einzelketten-Fv oder Diabody ist, der die in Seq.-ID Nr. 81 dargelegte VH-Domäne umfasst, mit der Ausnahme, dass die Aminosäure an Position 5 der VH-Domäne ein Leucin-Rest (L) anstelle eines Valin-Rests (V) ist, und der ferner die in Seq.-ID Nr. 82 dargelegte VL-Domäne umfasst, mit der Ausnahme, dass die Aminosäure an Position 18 der VL-Domäne ein Arginin-Rest (R) anstelle eines Lysin-Rests (K) ist.

15. In-vitro-Verfahren zur Diagnose eines Lymphoms, worin ein Antikörper, der die ED-A von Fibronectin bindet, verwendet wird.

## Revendications

1. Anticorps qui se lie à l'isoforme extra domaine-A (ED-A) de la fibronectine destiné à être utilisé dans un procédé de traitement d'un lymphome, où l'anticorps est conjugué à une molécule qui possède une activité biocide ou cytotoxique, ou à un radio-isotope.

2. Anticorps qui se lie à l'isoforme ED-A de la fibronectine destiné à être utilisé dans un procédé de détection ou de diagnostic in vivo d'un lymphome chez un humain ou un animal, où l'anticorps est marqué avec un marqueur détectable.

3. Anticorps destiné à être utilisé selon la revendication 2, où le procédé de détection ou de diagnostic d'un lymphome chez un humain ou un animal comprend les étapes qui consistent à :
(a) administrer à l'humain ou à l'animal un anticorps qui se lie à l'isoforme ED-A de la fibronectine, et
(b) déterminer la présence ou l'absence de l'anticorps dans le système lymphatique de l'organisme humain ou animal ;
où la localisation de l'anticorps dans le système lymphatique chez l'humain ou l'animal indique la présence d'un lymphome.

4. Anticorps qui se lie à l'isoforme ED-A de la fibronectine destiné à être utilisé dans un procédé de délivrance d'une molécule conjuguée à l'anticorps au système néovasculaire d'un lymphome chez un humain ou un animal, où la molécule possède une activité biocide ou cytotoxique, est un marqueur détectable, ou est un radio-isotope.

5. Utilisation d'un anticorps qui se lie à l'isoforme ED-A de la fibronectine pour la préparation d'un médicament destiné à une délivrance, à un lymphome, d'une molécule conjuguée à l'anticorps, où la molécule possède une activité biocide ou cytotoxique, est un marqueur détectable, ou est un radio-isotope.

6. Anticorps destiné à être utilisé selon la revendication 3, où l'anticorps est marqué avec un marqueur détectable.

7. Anticorps destiné à être utilisé selon l'une quelconque des revendications 1 à 4 ou 6, ou utilisation selon la revendication 5, où l'anticorps se lie à l'ED-A de la fibronectine.

8. Anticorps destiné à être utilisé selon l'une quelconque des revendications 1 à 4 ou 6 à 7, ou utilisation selon la revendication 5 ou 7, où le lymphome est un lymphome Hodgkinien ou un lymphome non Hodgkinien.

9. Anticorps destiné à être utilisé selon l'une quelconque des revendications 1 à 4 ou 6 à 8, ou utilisation selon la revendication 5, 7 ou 8, où l'anticorps comprend un domaine VH et un domaine VL, où le domaine VH comprend une charpente et un ensemble de régions déterminant la complémentarité HCDR1, HCDR2 et HCDR3, et où le domaine VL comprend un ensemble de régions déterminant la complémentarité LCDR1, LCDR2 et LCDR3 et une charpente, où :
HCDR1 possède la séquence d'acides aminés SEQ ID NO: 83,
HCDR2 possède la séquence d'acides aminés SEQ ID NO: 4,
HCDR3 possède la séquence d'acides aminés SEQ ID NO: 5 ;
LCDR1 possède la séquence d'acides aminés SEQ ID NO: 86,
LCDR2 possède la séquence d'acides aminés SEQ ID NO: 7, et
LCDR3 possède la séquence d'acides aminés SEQ ID NO: 8.

10. Anticorps destiné à être utilisé selon la revendication 9, ou utilisation selon la revendication 9, où la charpente du domaine VH et/ou la charpente du domaine VL est une charpente de lignée germinale humaine.

11. Anticorps destiné à être utilisé selon la revendication 9 ou 10, ou utilisation selon la revendication 9 ou 10, où l'anticorps comprend le domaine VH montré dans SEQ ID NO: 81 ; ou comprend le domaine VH montré dans SEQ ID NO: 81 sauf que l'acide aminé à la position 5 du domaine VH est un résidu leucine (L) plutôt qu'un résidu valine (V).

12. Anticorps destiné à être utilisé selon l'une quelconque des revendications 9 à 11, ou utilisation selon l'une quelconque des revendications 9 à 11, où l'anticorps comprend le domaine VL montré dans SEQ ID NO: 82 ; ou comprend le domaine VL montré dans SEQ ID NO: 82 sauf que l'acide aminé à la position 18 du domaine VL est un résidu arginine (R) plutôt qu'un résidu lysine (K).

13. Anticorps destiné à être utilisé selon l'une quelconque des revendications 1 à 4 ou 6 à 12, ou utilisation selon l'une quelconque des revendications 5 ou 7 à 12, où l'anticorps est un Fv simple chaîne ou un dianticorps.

14. Anticorps destiné à être utilisé selon l'une quelconque des revendications 1 à 4 ou 6 à 13, ou utilisation selon l'une quelconque des revendications 5 ou 7 à 13, où l'anticorps est un Fv simple chaîne ou un dianticorps comprenant le domaine VH montré dans SEQ ID NO: 81 sauf que l'acide aminé à la position 5 du domaine VH est un résidu leucine (L) plutôt qu'un résidu valine (V), et comprenant en outre le domaine VL montré dans SEQ ID NO: 82 sauf que l'acide aminé à la position 18 du domaine VL est un résidu arginine (R) plutôt qu'un résidu lysine (K).

15. Procédé de diagnostic *in vitro* d'un lymphome, où un anticorps qui se lie à l'ED-A de la fibronectine est utilisé.
